# EUROPEAN PATENT APPLICATION

(11) **EP 4 306 045 A1**
(43) Date of publication of application: **17.01.2024**
(21) Application number: 22767277.1
(22) Date of filing: 11.03.2022
(51) Int. Cl.: A61B 5/0531, A61B 5/16

(54) **SKIN RESISTANCE MEASUREMENT DEVICE, SKIN RESPONSIVENESS ASSESSMENT METHOD, AND BEHAVIOR/EMOTION DETERMINATION METHOD**

(30) Priority: 11.03.2021 JP 2021039017
(71) Applicant: The University of Tokyo, Bunkyo-ku, Tokyo 113-8654 (JP); Keio University, Tokyo, 108-8345 (JP)
(72) Inventor: SOMEYA Takao, Tokyo 113-8654 (JP); MIYAMOTO Akihito, Tokyo 113-8654 (JP); KAWASHIMA Ikue, Tokyo 113-8654 (JP); AMAGAI Masayuki, Tokyo 160-8582 (JP); KAWASAKI Hiroshi, Tokyo 160-8582 (JP)
(74) Representative: Ernest Gutmann - Yves Plasseraud S.A.S.
(86) International application number: PCT/JP2022/010989
(87) International publication number: WO 2022/191323

(57) **Abstract**

A skin resistance measurement device includes a pair of electrode portions provided on an epidermis and a measurement portion configured to measure skin resistance between the pair of electrode portions. The pair of electrode portions has a structure in which drying of the epidermis is naturally performed.

## Description

### [Technical Field]

The present invention relates to a skin resistance measurement device, a skin responsiveness assessment method, and a behavior/emotion determination method.

Priority is claimed on Japanese Patent Application No. 2021-039017, filed March 11, 2021, the content of which is incorporated herein by reference.

### [Background Art]

In recent years, flexible electronics have attracted much attention because they can be applied in various fields due to the softness of materials. In particular, interest in the healthcare field is increasing with the aging of the global society. For example, the flexible electronics are attracting attention as means for obtaining biological information directly from cells and tissues by attaching the flexible electronics to the surface of a human body or inside the body. As an effective means, electrodes capable of being directly attached to skin (for example, Patent Document 1) have attracted attention.

### [Citation List]

### [Patent Document]

[Patent Document 1]
PCT International Publication No. WO 2020/179907

### [Summary of Invention]

### [Technical Problem]

When electrodes are directly attached to the skin and skin resistance is measured over time, natural water transpiration from the skin may be blocked. Also, when the electrodes are attached for a long period of time, they may have an adverse influence of inflammation or the like on the skin. When electrodes are directly attached to the skin, it is necessary to perform measurement for a long period of time without blocking the natural water transpiration from the skin. In particular, it is possible to assess responsiveness of the skin to a change in an external environment and life behavior by assessing a rate of change in skin resistance. It is known that the skin is an organ that is affected by the external environment all the time and environmental changes can exacerbate diseases and change the health of the skin.

The present invention has been made in view of the above-described circumstances and provides a skin resistance measurement device, a skin responsiveness assessment method, and a behavior/emotion determination method capable of performing measurement for a long period of time without blocking natural water transpiration from a skin when electrodes are directly attached to the skin.

### [Solution to Problem]

In order to achieve the aforementioned objective, according to an aspect of the present invention, there is provided a skin resistance measurement device including: a pair of electrode portions to be provided on an epidermis; and a measurement portion configured to measure skin resistance between the pair of electrode portions, wherein the pair of electrode portions has a structure in which drying of the epidermis is naturally performed.

Also, according to the aspect of the present invention, in the skin resistance measurement device, the electrode portion has a fiber network.

Also, according to the aspect of the present invention, in the skin resistance measurement device, the electrode portion has fine linear members arranged at prescribed intervals.

Also, according to the aspect of the present invention, in the skin resistance measurement device, the electrode portion has a stripe-shaped opening.

Also, according to the aspect of the present invention, in the skin resistance measurement device, the electrode portion is a foil-shaped electrode with a crack or a pinhole.

Also, according to the aspect of the present invention, in the skin resistance measurement device, the electrode portion has a structure that releases moisture absorbed on a surface in contact with the epidermis.

Also, according to an aspect of the present invention, there is provided a skin resistance measurement device including: a pair of electrode portions to be provided on an epidermis; and a measurement portion configured to measure skin resistance between the pair of electrode portions, wherein the electrode portion includes an epidermis contact portion that is in contact with the epidermis and has a structure in which drying of the epidermis is naturally performed and a connection portion configured to electrically connect the electrode portion and the measurement portion.

Also, according to the aspect of the present invention, in the skin resistance measurement device, the connection portion has breathability.

Also, according to the aspect of the present invention, in the skin resistance measurement device, an area of a part in contact with the connection portion is smaller than an area of a part not in contact with the connection portion in a surface of the epidermis contact portion.

Also, according to the aspect of the present invention, in the skin resistance measurement device, a conductive member having breathability is inserted between the electrode portion and the connection portion.

Also, according to the aspect of the present invention, in the skin resistance measurement device, the electrode portion further includes an epidermis non-contact portion not in contact with the epidermis, the epidermis non-contact portion and the connection portion have a mutually overlapping region, and the skin resistance measurement device further includes an insulation portion configured to electrically insulate the epidermis non-contact portion and the epidermis in the region.

Also, according to the aspect of the present invention, the skin resistance measurement device further includes a detection circuit configured to detect whether or not the electrode portion has been connected to the connection portion.

Also, according to an aspect of the present invention, there is provided a skin resistance measurement device including: a pair of electrode portions to be provided on an epidermis; and a measurement portion configured to measure skin resistance between the pair of the electrode portions by applying a direct current (DC) voltage to the pair of electrode portions, wherein the pair of electrode portions has a structure in which drying of the epidermis is naturally performed.

Also, according to the aspect of the present invention, in the skin resistance measurement device, the measurement portion measures the skin resistance after applying the DC voltage to the pair of electrode portions for a first period of time that is a prescribed period of time in a period before the skin resistance is measured and pauses a process of applying the DC voltage to the pair of electrode portions for a second period of time that is a prescribed period of time.

Also, according to an aspect of the present invention, there is provided a skin responsiveness assessment method including: a measurement step of measuring skin resistance between a pair of electrode portions provided on an epidermis of a measurement target person and having a structure in which drying of the epidermis is naturally performed; and an assessment step of assessing responsiveness of the skin to an external environment and a change in life behavior of the measurement target person on the basis of the skin resistance measured in the measurement step.

Also, according to an aspect of the present invention, there is provided a behavior/emotion determination method including: a measurement step of measuring skin resistance between a pair of electrode portions provided on an epidermis of a measurement target person and having a structure in which drying of the epidermis is naturally performed; and a determination step of determining behavior or an emotion of the measurement target person on the basis of the skin resistance measured in the measurement step.

### [Advantageous Effects of Invention]

According to the present invention, it is possible to perform measurement for a long period of time without blocking natural water transpiration from skin when electrodes are directly attached to the skin.

### [Brief Description of Drawings]

FIG. 1 is a diagram showing an example of the appearance of a skin resistance measurement device attached to a skin according to an embodiment of the present invention.
FIG. 2 is a diagram showing an example of a configuration of the skin resistance measurement device according to the embodiment of the present invention.
FIG. 3 is a diagram showing an example of a cross-sectional view of the skin resistance measurement device according to the embodiment of the present invention.
FIG. 4 shows an example of a relationship between a surface area of an electrode and skin resistance according to the embodiment of the present invention.
FIG. 5 is a diagram showing an example of a cross-sectional view of the skin resistance measurement device according to a modified example of the embodiment of the present invention.
FIG. 6 is a diagram showing an example of a configuration of the skin resistance measurement device according to the modified example of the embodiment of the present invention.
FIG. 7 shows an example of the appearance of the skin resistance measurement device attached to the skin according to the modified example of the embodiment of the present invention.
FIG. 8 is a diagram showing an example of a cross-sectional view of the skin resistance measurement device according to the modified example of the embodiment of the present invention.
FIG. 9 is a diagram showing an example of the cross-sectional view of the skin resistance measurement device according to the modified example of the embodiment of the present invention.
FIG. 10 is a diagram showing an example of a pattern in which a direct current (DC) voltage is applied according to the embodiment of the present invention.
FIG. 11 is a diagram showing an example of a result of measuring a change over time in skin resistance according to a first embodiment example of the present invention.
FIG. 12 is a diagram showing an example of the appearance of a skin resistance measurement device attached to an epidermis when electrodes were covered with a film cover according to the first embodiment example of the present invention.
FIG. 13 is a diagram showing an example of a result of measuring the change over time in the skin resistance according to the first embodiment example of the present invention.
FIG. 14 is a diagram showing an example of the change over time in the skin resistance obtained by extracting a measurement result for a period of 1 hour to 2 hours from the start of measurement among measurement results according to the first embodiment example of the present invention.
FIG. 15 is a diagram showing an example of the change in the skin resistance that occurs when the film is attached and detached according to the first embodiment example of the present invention.
FIG. 16 is a diagram showing an example of a drying process of sweating due to exercise according to the first embodiment example of the present invention.
FIG. 17 is a diagram showing an example of the appearance of a skin resistance measurement device attached to an epidermis when electrodes were covered with an adhesive plaster according to a second embodiment example of the present invention.
FIG. 18 is a diagram showing an example of a result of measuring a change over time in skin resistance when an adhesive plaster was attached to the epidermis according to the second embodiment example of the present invention.
FIG. 19 is a diagram showing an example of a measurement result for verifying the reproducibility of an influence on skin resistance when the adhesive plaster was attached to the epidermis according to the second embodiment example of the present invention.
FIG. 20 is a diagram showing an example of a measurement result for verifying the reproducibility of an influence on skin resistance when a polyurethane film was attached to the epidermis according to the second embodiment example of the present invention.
FIG. 21 is a diagram showing an example of a result of measuring skin resistance when the film cover was attached to the epidermis to cover the electrodes and removed according to the second embodiment example of the present invention.
FIG. 22 is a diagram showing an example of a result of measuring a transepidermal water transpiration amount using a skin measurement instrument to be compared according to the second embodiment example of the present invention.
FIG. 23 is a diagram showing an example in which the result of measuring skin resistance using the skin resistance measurement device according to the second embodiment example of the present invention is compared with the result of measuring the transepidermal water transpiration amount using the skin measurement instrument to be compared.
FIG. 24 is a diagram showing an example of the result of calculating a correlation coefficient for a correlation between the skin resistance and the transepidermal water transpiration amount according to the second embodiment example of the present invention.
FIG. 25 is a diagram showing an example of the result of measuring a water content of a stratum corneum using the skin measurement instrument to be compared according to the second embodiment example of the present invention.
FIG. 26 is a diagram showing an example of a result of measuring skin resistance when polyethylene was used as a film cover with which the electrodes were covered according to a third embodiment example of the present invention.
FIG. 27 is a diagram showing an example of a result of measuring skin resistance when polyurethane was used as the film cover with which the electrodes were covered according to the third embodiment example of the present invention.
FIG. 28 is a diagram showing an example of a measurement result when the measurement target person was a man in his 60s and polyethylene was used as the film cover according to the third embodiment example of the present invention.
FIG. 29 is a diagram showing an example of a measurement result when the measurement target person was the man and polyurethane was used as the film cover according to the third embodiment example of the present invention.
FIG. 30 is a diagram showing an example of a measurement result when the measurement target person was a woman in her 40s and polyethylene was used as the film cover according to the third embodiment example of the present invention.
FIG. 31 is a diagram showing an example of a result of enlarging a certain range of the measurement result of FIG. 30 in the direction of the skin resistance value according to the third embodiment example of the present invention.
FIG. 32 is a diagram showing an example of a result of measuring skin resistance according to a comparative example of a fourth embodiment example of the present invention.
FIG. 33 is a diagram showing an example of a result of measuring skin resistance according to the comparative example of the fourth embodiment example of the present invention.
FIG. 34 is a diagram showing an example of a result of measuring skin resistance according to the fourth embodiment example of the present invention.

### [Description of Embodiments]

### (Embodiments)

Hereinafter, embodiments of the present invention will be described in detail with reference to the drawings.

### [Configuration of skin resistance measurement device]

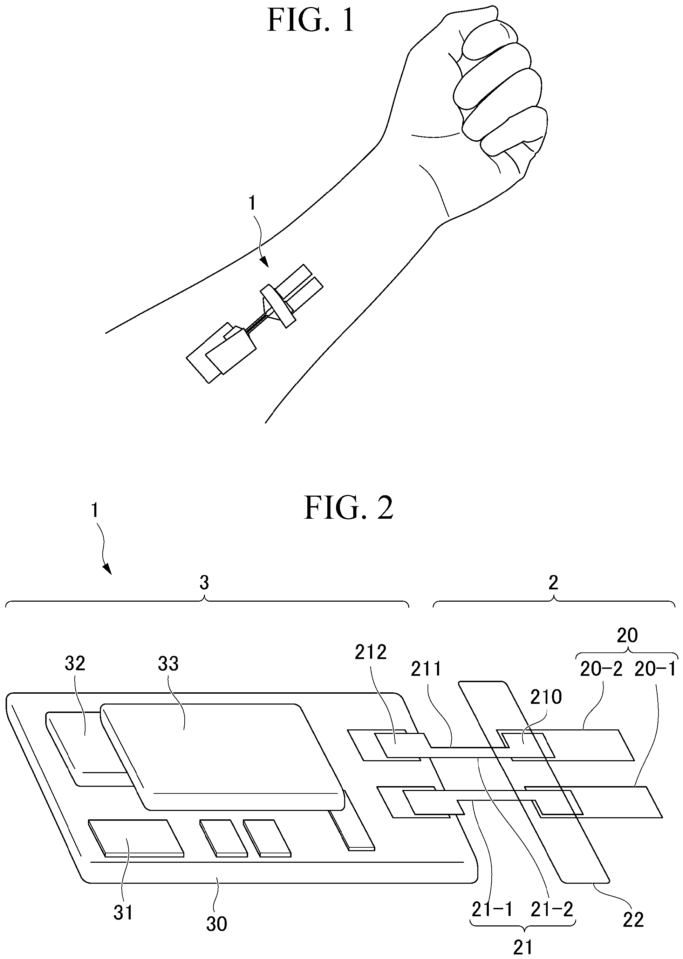

A configuration of a skin resistance measurement device 1 according to the present embodiment will be described with reference to FIGS. 1 to 3. FIG. 1 is a diagram showing an example of the appearance of the skin resistance measurement device 1 attached to the skin according to the present embodiment. As shown in FIG. 1, the skin resistance measurement device 1 is directly attached to the skin of a measurement target person and measures skin resistance. The skin resistance measurement device 1 is a wearable measurement device.

In general, skin resistance is resistance along a path on an epidermis from one position of the epidermis to another position of the epidermis and resistance along a path from one position of the epidermis to the other position of the epidermis through subcutaneous tissue. In the present embodiment, the skin resistance is mainly the latter. That is, in the present embodiment, the skin resistance is mainly resistance along a path from one position of the epidermis to another position of the epidermis through the subcutaneous tissue.

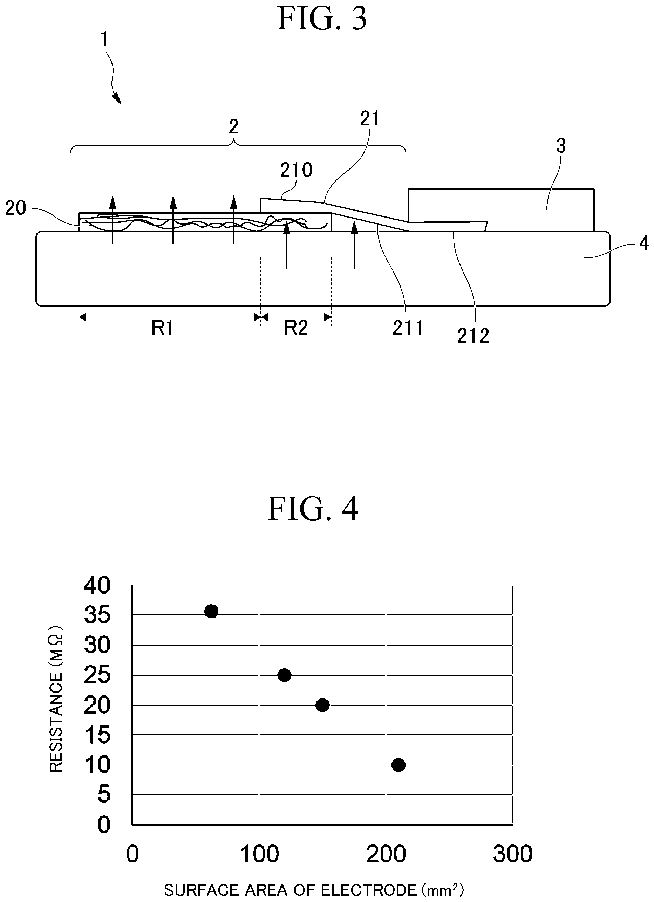

FIG. 2 is a diagram showing an example of a configuration of the skin resistance measurement device 1 according to the present embodiment. FIG. 3 is a diagram showing an example of a cross-sectional view of the skin resistance measurement device 1 according to the present embodiment. The skin resistance measurement device 1 includes an electrode portion 2 and a measurement portion 3.

The electrode portion 2 is provided on an epidermis 4. The electrode portion 2 includes electrodes 20 and a connection portion 21. The electrodes 20 include a pair of electrodes 20-1 and 20-2.

Because the electrode 20-1 and the electrode 20-2 have the same configuration, the electrode 20-1 and the electrode 20-2 will be referred to as the electrode 20 when they are not distinguished in the following description.

The electrodes 20 are provided in contact with the epidermis 4. The shape of the electrode 20 is not particularly limited and may be rectangular, round, or comb-shaped. The round or comb-shaped shape is generally used as the shape of electrodes provided in commercially available electrodermal activity (EDA) and skin moisture measurement devices. However, because anyone needs to easily use the electrodes 20 when the electrodes 20 are actually used, a configuration as simple as possible is highly important for practical use. As an example, it is a plate-shaped or foil-shaped (film-shaped) rectangle. The shape of the electrode 20 is also referred to as a zonal shape. The size of the electrode 20 is a size for covering one or more sweat glands on the epidermis 4. As an example, the size of the electrode 20 is about 6 mm on the short side and about 24 mm to 35 mm on the long side. As an example, the spacing between the electrode 20-1 and the electrode 20-2 is about 1 mm to 2 mm. Also, the current path passing over the epidermis may be generated due to severe sweating or the like and a spacing between the electrode 20-1 and the electrode 20-2 may be 2 mm or more as necessary for the purpose of preventing the generation of an electric current path.

The electrode 20 has a fiber network made of nanofibers as an example. In other words, the electrode 20 has a nanomesh structure. As an example, the electrode 20 includes an electrode layer formed by a nanofiber network made of water-soluble polyvinyl alcohol (PVA) in an electrospinning method, and gold deposited thereon. The nanofibers constituting the fiber network of the electrode 20 have a diameter of 200 nm to 2 µm. Also, the thickness of the deposited gold film is 10 nm to 200 nm.

The above-described nanomesh structure is provided in the electrode 20, surface followability, lateral stretchability, gas and moisture permeability, and transparency are enhanced.

The electrode 20 has a nanomesh structure, thereby having breathability. The breathability indicates that gas and moisture permeability are high. Therefore, when water evaporates from sweat secreted from sweat glands distributed on the epidermis 4 with which the electrode 20 is in contact or from the stratum corneum of the skin, their water evaporation amounts are close to an evaporation amount of a case where the electrode 20 is not provided. That is, because the electrode 20 has a nanomesh structure, drying of the epidermis 4 with which the electrode 20 is in contact is naturally performed.

Thus, the electrode 20 has a structure in which drying of the epidermis 4 to naturally performed. The fact that drying of the epidermis 4 is naturally performed is that it has higher breathability than, for example, polyurethane and the like. The breathability is, for example, about 1000 g/m²·24h to 20000 g/m²·24h. The electrode 20 is an example of an epidermal contact portion provided in the electrode portion.

Also, the electrode 20 may have a structure other than the nanomesh structure as a structure in which drying of the epidermis 4 is naturally performed. Here, another example of a structure in which drying of the epidermis 4 is naturally performed is given.

The electrode 20 may have a structure in which a plurality of fine linear members such as nanofibers are arranged substantially in parallel. Alternatively, the electrode 20 may have a lattice structure formed by arranging the plurality of linear members substantially orthogonally. That is, the electrode 20 may have fine linear members arranged at prescribed intervals. When the electrode 20 has fine linear members arranged at prescribed intervals, a spacing between two or more linear members is wider than, for example, the diameter of the sweat gland. Even if the spacing between the two or more linear members is narrower than the diameter of the sweat gland, the spacing is widely provided so that an opening ratio of the surface in contact with the epidermis 4 of the electrode 20 is higher than or equal to the prescribed ratio. The width of the fine linear member is, for example, 1 mm or less. The fine linear member is arranged so that the opening ratio is, for example, 50%.

Also, the electrode 20 may have a stripe-shaped opening. In this case, the opening is a rectangular shape as an example. An area of the opening and a spacing between adjacent openings are widely provided so that the opening ratio of the surface in contact with the epidermis 4 of the electrode 20 is higher than or equal to a prescribed ratio. The area of the opening and the spacing between the adjacent openings are arranged so that the opening ratio is, for example, 50%.

Also, the electrode 20 may be a foil-shaped electrode containing cracks. In this case, the cracks are formed in the electrode 20 by setting a width of the crack, an average spacing between the cracks, and the number of cracks so that the opening ratio of the surface in contact with the epidermis 4 of the electrode 20 is higher than or equal to a prescribed ratio. The foil-shaped electrode is, for example, gold leaf.

Also, the electrode 20 may have a plurality of holes as a structure in which drying of the epidermis 4 is naturally performed. In this case, for example, the electrode 20 may be a foil-shaped electrode with a plurality of pinholes. The plurality of pinholes are provided in the foil-shaped electrode by setting a diameter of each of the plurality of pinholes, an average spacing between the pinholes, and the number of pinholes so that the opening ratio of the surface in contact with the epidermis 4 of the electrode 20 is higher than or equal to the prescribed ratio. For example, the diameter of the pinhole is several tens of micrometers (µm) to several hundreds of micrometers (µm), and the average spacing between the pinholes is 0.1 mm to 1 mm.

In any of the above cases, it is preferable not to cover each sweat gland at a prescribed proportion or more by providing the electrode 20 with a gap of a prescribed size or more so that the electrode 20 has a structure with breathability or the like in which drying of the epidermis 4 is naturally performed. That is, a surface area in which the electrode 20 is in contact with the epidermis 4 is preferably a prescribed size or less. On the other hand, when the surface area of the electrode 20 becomes smaller, because the contact resistance of the electrode 20 increases, a current does not easily flow between the electrode 20-1 and the electrode 20-2 and the skin resistance measurement itself becomes difficult. For this reason, the size of the gap provided in the electrode 20 is preferably within a prescribed range. The opening ratio of the surface in contact with the epidermis 4 of the electrode 20 is, for example, 10% or more. More preferably, the opening ratio is 30% or more.

FIG. 4 shows an example of a relationship between the surface area of the electrode 20 and skin resistance according to the present embodiment. Although a value calculated from the outer shape of the electrode 20 as the surface area of the electrode 20 is shown in FIG. 4, an actual surface area of the electrode is calculated by multiplying the surface area of the outer shape of the electrode by an occupancy ratio of the electrode in the outer shape.

Also, the electrode 20 has a structure that releases moisture absorbed on a surface in contact with the epidermis 4 even if moisture (including sweat) evaporated from the skin is absorbed as a structure in which drying of the epidermis 4 is naturally performed. For example, the electrode 20 releases the moisture absorbed on the surface in contact with the epidermis 4 from a surface on the opposite side of the surface. When the electrode 20 has a structure that releases moisture absorbed on the surface in contact with the epidermis 4, the material of the electrode 20 is, for example, a water retention material used for the skin. The water retention material is, for example, polyvinyl alcohol, modified polyvinyl alcohol, sodium polyacrylate, collagen, or glycerin.

Description of a configuration of the skin resistance measurement device 1 continues.

The connection portion 21 electrically connects the electrodes 20 and the measurement portion 3. The connection portion 21 includes a connection portion 21-1 and a connection portion 21-2. The connection portion 21-1 electrically connects the electrode 20-1 and the measurement portion 3. The connection portion 21-2 electrically connects the electrode 20-2 and the measurement portion 3.

Because the connection portion 21-1 and the connection portion 21-2 have the same configuration, the connection portion 21-1 and the connection portion 21-2 are referred to as the connection portion 21 when they are not distinguished in the following description.

The connection portion 21 is molded into a thin film shape. The connection portion 21 includes an electrode contact portion 210, a connection portion 211, and a connector contact portion 212. Although the electrode contact portion 210, the connection portion 211, and the connector contact portion 212 are shown only for one of the connection portions 21-2 in FIG. 2, a configuration including the electrode contact portion 210, the connection portion 211, and the connector contact portion 212 are provided for the connection portion 21-1 like the connection portion 21-2. The electrode contact portion 210 comes into contact with the surface of the electrode 20. The connector contact portion 212 comes into contact with the connector provided in the measurement portion 3. The connection portion 211 connects the electrode contact portion 210 and the connector contact portion 212. The electrode contact portion 210 and the connector contact portion 212 have a thin plate-like shape.

The electrode contact portion 210 is in contact with the electrode 20 from the upper side. Also, the electrode contact portion 210 may bias the electrode 20 from the upper side to the side of the epidermis 4 by a biasing force of the connection portion 211. Here, the upper side is the surface side of the electrode 20 not in contact with the epidermis 4. An adhesive portion 22 is attached to the epidermis 4 as well as the electrode contact portion 210 in the connection portion 21. In the present embodiment, as an example, the electrode contact portion 210 is attached to the epidermis 4 for each of the connection portion 21-1 and the connection portion 21-2 through one adhesive portion 22.

Although the adhesive portion 22 is attached to the electrode 20 from the top of the electrode contact portion 210 in FIG. 2, it may be separately provided between the electrode 20-1 and the electrode contact portion 210, and between the electrode 20-2 and the electrode contact portion 210 when a highly conductive adhesive is used. Also, for the purpose of preventing the contact between the electrode 20 and the electrode contact portion 210 from deteriorating due to body movement or the like, a material having conductivity and elasticity in upward and downward directions such as a sponge coated with a carbon material having high conductivity is provided between the electrode 20 and the electrode contact portion 210. Here, coating the sponge with a certain substance is attaching the substance to each surface of fine fibers constituting the sponge. Furthermore, for the purpose of increasing the pressure pressed by the electrode contact portion 210 against the electrode 20, a material having high elasticity such as a sponge may be provided between the adhesive portion 22 and the electrode contact portion 210.

The adhesive portion 22 suppresses the electrode 20 and the electrode contact portion 210 from being removed from the epidermis 4. It is only necessary for the material of the adhesive portion 22 capable of being attached to the epidermis 4 for a long period of time with adhesion to be a material that does not easily have an influence of inflammation or the like on the epidermis 4. The adhesive portion 22 preferably has breathability. The adhesive portion 22 is a surgical tape for medical use as an example.

Although the connector contact portion 212 is connected on a surface on the opposite side of the epidermis on the measurement portion 3 in FIG. 2, the connector contact portion 212 may be connected between the epidermis and the measurement portion 3 as shown in FIG. 3. Likewise, although the electrode contact portion 210 is connected on a surface on the opposite side of the surface where the electrode 20 comes into contact with the epidermis in FIG. 2, the electrode contact portion 210 may be connected between the epidermis and the electrode 20.

Although the connection portion 21 is used as wiring to electrically connect the electrode 20 and the measurement portion 3 in FIG. 2, the electrode 20 may be electrically connected to the measurement portion 3 without using the connection portion 21.

In the cross-sectional view shown in FIG. 3, a breathable region R1 indicates a part of the surface of the electrode 20 not in contact with the electrode contact portion 210. A non-breathable region R2 indicates a part of the surface of the electrode 20 in contact with the electrode contact portion 210. As described above, the electrode 20 has breathability. On the other hand, in the present embodiment, the connection portion 21 does not have breathability. Thus, the electrode portion 2 has breathability in the breathable region R1, but the electrode portion 2 does not have breathability in the non-breathable region R2. In the present embodiment, an area of the non-breathable region R2 is smaller than an area of the breathable region R1 in the surface of the electrode portion 2. That is, the area of the part in contact with the connection portion 21 as an example of the skin contact portion is smaller than the area of the part not in contact with the connection portion 21 in the surface of the electrode 20. Thus, the skin resistance measurement device 1 can have higher breathability than when the area of the part of the surface of the electrode 20 in contact with the connection portion 21 is larger than the area of the part not in contact with the connection portion 21.

Also, the contact resistance between the electrode contact portion 210 and the electrode 20 is sufficiently less than the contact resistance between the epidermis 4 and the electrode 20.

The connection portion 21 may have a portion in contact with the epidermis 4 because it is covered with an insulation material other than the connection portion. For example, a part of the connection portion 211 may be in contact with the epidermis 4.

A width of the connection portion 211 is narrower than those of the electrode contact portion 210 and the connector contact portion 212. Thereby, when an external force is applied to the skin resistance measurement device 1, the connection portion 211 absorbs an external force by flexing and the skin resistance measurement device 1 and the electrode 20 are prevented from being damaged.

As an example, the connection portion 21 is formed in plating or vapor deposition of gold on the surface of copper formed on a polyurethane film. Also, the material constituting the connection portion 21 may have conductivity. For example, metals such as copper, gold, aluminum, silver, and zinc can be used. From the viewpoint of conductivity, copper and silver are particularly preferable. When the connection portion 21 has a portion in contact with the epidermis 4, it is preferable to use stable gold to suppress an unnecessary reaction. The connection portion 21 may be polyurethane having conductivity.

More specifically, the connection portion 21 has copper wiring formed on a polyurethane film and is covered with a polyurethane film on the upper part thereof. Also, the connection portion 21 is opened in the portion of the electrode contact portion 210 and the connector contact portion 212 and has the top surface plated with gold. For this reason, a portion other than the electrode contact portion 210 and the connector contact portion 212 among portions constituting the connection portion 21 is electrically insulated.

The measurement portion 3 measures the skin resistance between the electrode 20-1 and the electrode 20-2. Here, the measurement portion 3 measures the skin resistance between the electrode 20-1 and the electrode 20-2 by applying a DC voltage between the electrode 20-1 and the electrode 20-2.

In the skin resistance measurement device 1, the DC voltage is applied. When an alternating current (AC) voltage is applied, the impedance including a capacitance component and a resistance component of the skin is measured and the DC resistance of the skin cannot be directly measured. In the skin resistance measurement device 1, the skin resistance value can be directly measured by applying a DC voltage, unlike a case where the AC voltage is applied.

The measurement portion 3 includes a substrate 30, a switch 31, a memory 32, and a battery 33.

The substrate 30 is arranged in a state in which various types of components including the switch 31, the memory 32, the battery 33, and an arithmetic circuit IC (not shown) for measuring the resistance value are electrically connected. The substrate 30 is provided in contact with the epidermis 4. As an example, the substrate 30 has a size of about 23 mm in length × 16 mm in width. Because the skin resistance measurement device 1 is a wearable measurement device, the size of the substrate 30 is preferably small. If the size of the substrate 30 is excessively large, it becomes difficult to attach the substrate 30 to the epidermis 4. As an example, the substrate 30 is a film substrate such as a polyimide film. Because the substrate 30 is a film substrate, it is possible to ensure ease of attachment at the time of attachment to the epidermis 4 or followability to skin deformation.

The switch 31 switches a state of a power supply of the measurement portion 3 between an ON state and an OFF state. When the switch 31 is in the ON state, the measurement portion 3 measures skin resistance.

The memory 32 stores a time-series pattern between the ON state and the OFF state of the voltage applied to the electrode 20. Also, the memory 32 stores the skin resistance value measured by the measurement portion 3 in a time series.

The battery 33 supplies electric power to the measurement portion 3 when the switch 31 is in the ON state.

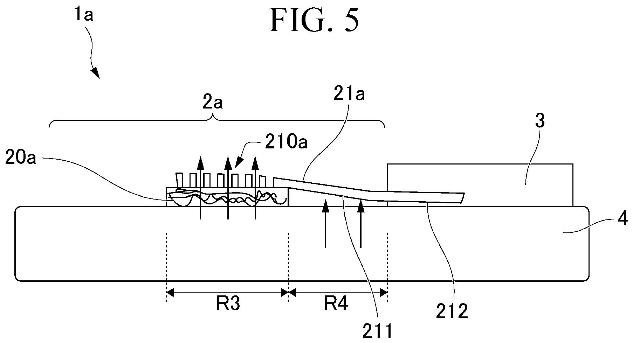

Also, the configuration of the skin resistance measurement device 1 is not limited to the configuration shown in FIG. 3. FIG. 5 is a diagram showing an example of a cross-sectional view of a skin resistance measurement device 1a according to a modified example of the present embodiment. A skin resistance measurement device 1a includes an electrode portion 2a and a measurement portion 3. The electrode portion 2a includes electrodes 20a and a connection portion 21a. The connection portion 21a includes an electrode contact portion 210a, a connection portion 211, and a connector contact portion 212.

The skin resistance measurement device 1a has the electrodes 20a and an electrode contact portion 210a different from those of the skin resistance measurement device 1 shown in FIG. 3. The configuration of the skin resistance measurement device 1a is similar to that of the skin resistance measurement device 1 shown in FIG. 3 in that the electrodes 20a are a pair of electrodes and the electrode contact portion 210a includes a pair of connection portions. The shape of the electrode 20a is different from the shape of the electrode 20 shown in FIG. 3 in that the long side of the rectangle is shorter. As an example, the surface area of the electrode contact portion 210a and the surface area of the electrode 20a are substantially equal.

Although the adhesive portion 22 shown in FIG. 2 is omitted in the cross-sectional view shown in FIG. 5, an adhesive member having high breathability is attached to the epidermis 4 as well as the electrode contact portion 210a within the connection portion 21a.

In the cross-sectional view shown in FIG. 5, a breathable region R3 indicates a portion in which the electrode 20a and the electrode contact portion 210a overlap. A non-breathable region R4 indicates the connection portion 211 within the connection portion 21a. The skin resistance measurement device 1a has breathability in the breathable region R3 and does not have breathability in the non-breathable region R4. Here, as in the connection portion 21 shown in FIG. 2, the connection portion 211 in the connection portion 21a is configured to have a width narrower than those of the electrode contact portion 210a and the connector contact portion 212a. Thus, in the non-breathable region R4 indicating the connection portion 21a, the accumulation of secretions such as sweat can be suppressed during measurement.

The electrode contact portion 210a has a structure in which a plurality of holes each having a prescribed diameter are provided as an example. The electrode contact portion 210a may have a mesh structure instead of the structure in which the plurality of holes each having the prescribed diameter are provided in a corresponding portion. In this portion, the opening ratio is higher than or equal to a prescribed ratio.

The electrode contact portion 210a has a structure in which drying of the epidermis is naturally performed. Thus, even if the electrode 20a and the electrode contact portion 210a overlap, an overlapping portion has breathability. The skin resistance measurement device 1a can compactly reduce a device size by a part where the electrode 20a and the electrode contact portion 210a overlap in a state in which there is a structure in which drying of the epidermis is naturally performed.

Although an example in which the surface area of the electrode contact portion 210a is substantially the same as the surface area of the electrode 20a has been described as an example in FIG. 5, the present invention is not limited thereto. The surface area of the electrode contact portion 210a may be smaller than the surface area of the electrode 20a. That is, the area of the part of the surface of the electrode 20a in contact with the connection portion 21a may be smaller than the area of the part not in contact with the connection portion 21a.

Although an example in which the connection portion 21 or the connection portion 21a is provided on the upper side of the electrode 20 (i.e., the surface of the electrode 20 is on the opposite side of the epidermis 4) has been described in the present embodiment, the present invention is not limited thereto. The connection portion 21 may be provided between the electrode 20 and the epidermis 4 (i.e., on the side of the epidermis 4 within the surface of the electrode 20).

Although an example in which the electrodes 20 are connected to the measurement portion 3 via the connection portion 21 has been described in the present embodiment, the present invention is not limited thereto. The electrode portion 2 does not include a connection portion 21 and the electrodes 20 may be directly connected to the measurement portion 3.

While the electrodes 20 are attached to the epidermis 4, the measurement portion 3 or both the measurement portion 3 and the connection portion 21 may be configured to be removable. For example, in measurement, the electrode 20 may remain attached to the epidermis 4 and only the measurement portion 3 may be replaced.

Although an example in which the connection portion 21 is a wiring material having no breathability has been described in the present embodiment, the present invention is not limited thereto. As the connection portion 21, for example, a wiring material in which electrodes formed in a mesh shape are used on a highly breathable polyurethane substrate having a thickness of 50 µm or less may be used. In this case, not only the electrodes 20 but also the connection portion 21 can have breathability. Furthermore, if a highly breathable adhesive member is used for the adhesive portion 22, breathability can also be ensured in the non-breathable region R2 shown in FIG. 3. Thus, skin resistance can be accurately measured even if the area of the region R2 is not sufficiently smaller than the area of the region R1. Also, when a highly breathable adhesive member is used for the adhesive portion 22, it is not necessary to make the area of the region R2 sufficiently smaller than the area of the region R1 and therefore it is possible to stably form connections between the electrodes 20 and the connection portion 21.

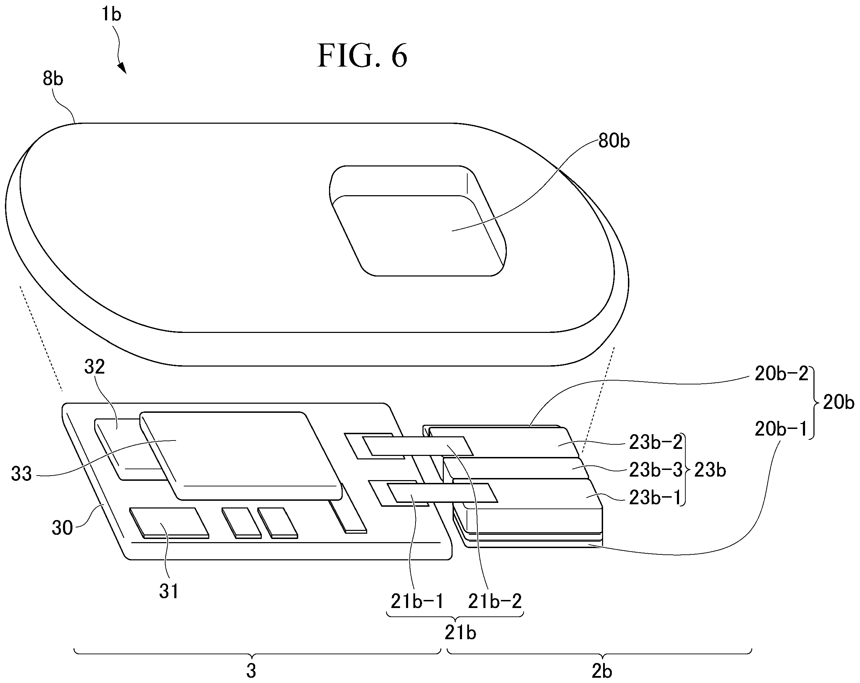

As a configuration of the skin resistance measurement device, a conductive member having breathability may be inserted between the electrode and the connection portion. A case where a conductive member having breathability is inserted between the electrode and the connection portion will be described as another modified example of the configuration of the skin resistance measurement device with reference to FIGS. 6 to 8.

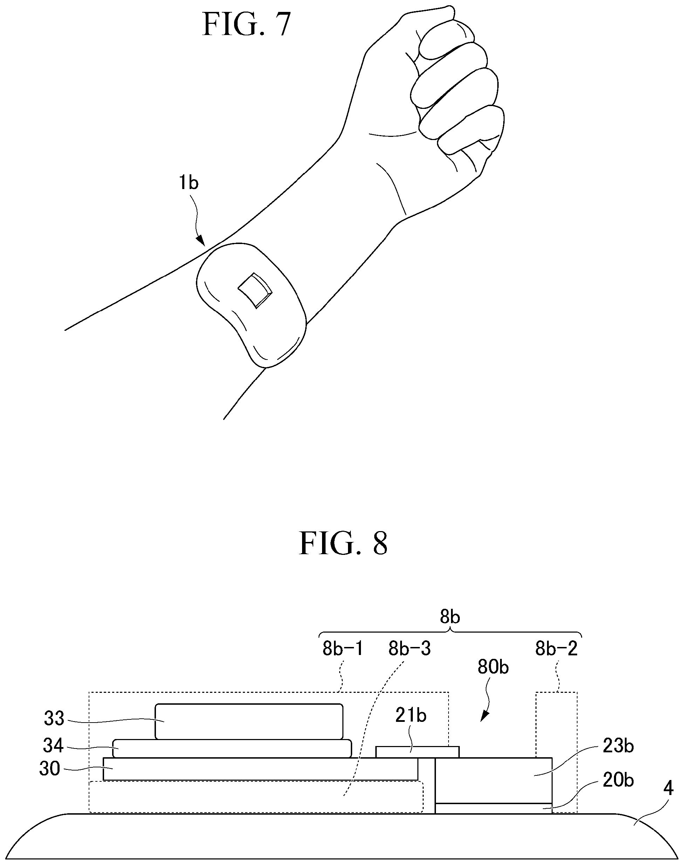

FIG. 6 is a diagram showing an example of a configuration of a skin resistance measurement device 1b according to the modified example of the present embodiment. FIG. 7 shows an example of the appearance of the skin resistance measurement device 1b attached to the skin according to the modified example of the present embodiment. FIG. 8 is a diagram showing an example of a cross-sectional view of the skin resistance measurement device 1b according to the modified example of the present embodiment. Also, in FIG. 8, components on the substrate 30, such as the switch 31 and the memory 32 shown in FIG. 6, are collectively shown as circuit components 34.

The skin resistance measurement device 1b includes an electrode portion 2b, a measurement portion 3, and a case 8b. Because the configuration of the measurement portion 3 shown in FIG. 6 is similar to the configuration of the measurement portion 3 shown in FIG. 2, description thereof will be omitted.

The electrode portion 2b includes electrodes 20b, a connection portion 21b, and a conductive sponge 23b.

The electrodes 20b include an electrode 20b-1 and an electrode 20b-2 that are a pair of electrodes. Also, because the electrode 20b-1 and the electrode 20b-2 have the same configuration, the electrode 20b-1 and the electrode 20-2b will be referred to as the electrode 20b when they are not distinguished in the following description.

The conductive sponge 23b is a member having breathability and conductivity. The conductive sponge 23b is made of a foam sponge such as polyurethane and a part of the foam sponge is assigned conductivity due to coating with a substance having conductivity. Coating the part of the foam sponge with the substance having conductivity is attaching the substance to each surface of the fine fibers constituting the part. The substance having conductivity is, for example, silver/silver chloride, silver, carbon, or the like.

The conductive sponge 23b includes a conductive portion 23b-1, a conductive portion 23b-2, and a non-conductive portion 23b-3. Each of the conductive portion 23b-1 and the conductive portion 23b-2 has conductivity. Each of the conductive portion 23b-1 and the conductive portion 23b-2 is a portion in which the foam sponge is coated with the substance having conductivity.

The non-conductive portion 23b-3 has no conductivity. The non-conductive portion 23b-3 includes a foam sponge and is not coated with the substance having conductivity. The non-conductive portion 23b-3 is arranged between the conductive portion 23b-1 and the conductive portion 23b-2 to insulate the conductive portion 23b-1 and the conductive portion 23b-2.

The conductive portion 23b-1 is arranged between the electrode 20b-1 and the connection portion 21b-1. Thereby, the conductive portion 23b-1 electrically connects the electrode 20b-1 and the connection portion 21b-1. The conductive portion 23b-2 is arranged between the electrode 20b-2 and the connection portion 21b-2. Thereby, the conductive portion 23b-2 electrically connects the electrode 20b-2 and the connection portion 21b-2.

That is, the connection portion 21b electrically connects the electrode 20b and the measurement portion 3 via the conductive portion 23b-1 and the conductive portion 23b-2.

The electrode portion 2b and the measurement portion 3 are covered with the case 8b. As shown in FIG. 7, in the skin resistance measurement device 1b, the measurement portion 3 is covered with the case 8b and therefore the measurement portion 3 is prevented from being exposed in a state in which the skin resistance measurement device 1b is attached to the skin.

A vent 80b is provided in the case 8b. Here, as shown in the cross-sectional view of the skin resistance measurement device 1b shown in FIG. 8, the case 8b is divided into three parts, i.e., a part 8b-1, a part 8b-2, and a part 8b-3. The vent 80b is provided between the part 8b-1 and the part 8b-2. In the case 8b with which the measurement portion 3 is covered, the conductive sponge 23b is exposed from the vent 80b. Thus, in the skin resistance measurement device 1b, even if the measurement portion 3 is covered with the case 8b, the electrode 20b has a structure in which drying of the epidermis 4 is naturally performed.

The part 8b-3 is a part in which the case 8b is in contact with the epidermis 4. The part 8b-3 has adhesiveness on the surface and can be attached to the epidermis 4. The part 8b-3 may include a seal. In this case, when the skin resistance measurement device 1b is attached to the epidermis 4, the mount of the seal provided by the part 8b-3 is removed and the part 8b-3 is attached to the epidermis 4.

Because the part 8b-3 has adhesiveness, the skin resistance measurement device 1b can be easily attached to the epidermis 4. In the skin resistance measurement device 1b, because the case 8b covers the electrode portion 2b and the measurement portion 3, the electrode portion 2b and the measurement portion 3 can be fixed to the epidermis 4 by attaching the case 8b to the epidermis 4.

The material of the case 8b may be any material as long as it has a soft material that does not easily damage the skin. Examples of the material of the case 8b are silicone, polyurethane having a thickness greater than or equal to a prescribed thickness, a foaming agent, and the like.

Also, as described in FIG. 3, in the skin resistance measurement device 1 according to the present embodiment, the electrode 20 does not have breathability in the non-breathable region R2, which is a part of the surface of the electrode 20 in contact with the electrode contact portion 210 of the connection portion 21. The skin resistance measurement device 1 according to the present embodiment has a higher breathable configuration in the case where the area of the part of the surface of the electrode 20 in contact with the connection portion 21 is smaller than the area of the part not in contact with the connection portion 21 than in the case where the area of the part of the surface of the electrode 20 in contact with the connection portion 21 is larger than the area of the part not in contact with the connection portion 21. That is, in the skin resistance measurement device 1 according to the present embodiment, an influence of the skin resistance of the portion that does not have breathability on the measurement result is minimized as much as possible by minimizing the portion that does not have breathability in the electrode 20 in contact with the skin.

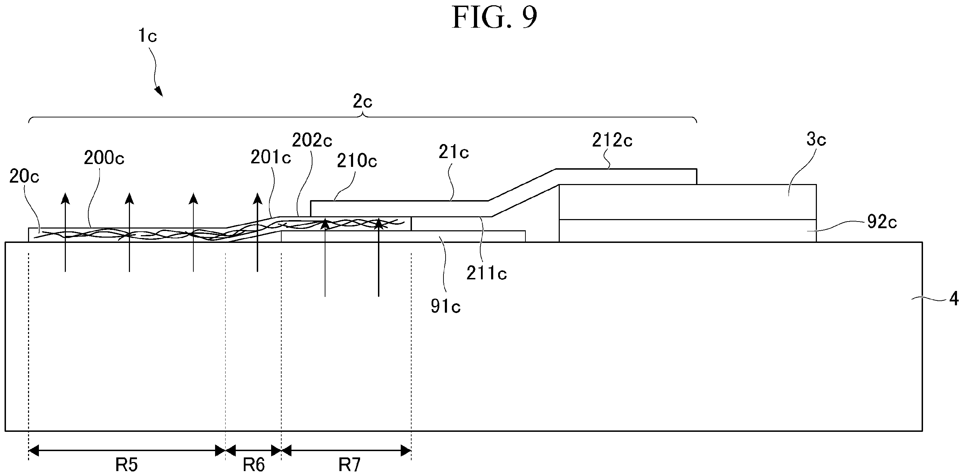

As a configuration of the skin resistance measurement device, the influence of the skin resistance of the non-breathable region on the measurement result may be further reduced by electrically insulating the electrode and the skin in a non-breathable region that is a part of the surface of the electrode that is in contact (overlapping) with the connection portion. A case where an insulation material for electrically insulating the electrode and the skin is provided between the electrode and the skin is provided in a portion of the electrode surface that is in contact with the electrode contact portion will be described as another modified example of the configuration of the skin resistance measurement device with reference to FIG. 9.

FIG. 9 is a diagram showing an example of a cross-sectional view of a skin resistance measurement device 1c according to a modified example of the present embodiment. The skin resistance measurement device 1c includes an electrode portion 2c, a measurement portion 3c, an adhesive tape 91c, and a double-sided tape 92c. The electrode portion 2c includes electrodes 20c and a connection portion 21c. The configuration of the skin resistance measurement device 1c is similar to that of the skin resistance measurement device 1 shown in FIG. 3 in that the electrodes 20c are a pair of electrodes and the connection portion 21c is made of a pair of connection portions.

A shape of the electrode 20c is different from that of the electrode 20 shown in FIG. 3. Like the electrode 20, the electrode 20c has a structure in which drying of the epidermis 4 is naturally performed, such as a nanomesh structure.

The electrode 20c includes a skin contact portion 200c, a connection portion 201c, and a tape contact portion 202c. The skin contact portion 200c, the connection portion 201c, and the tape contact portion 202c each have a thin plate-like shape.

The skin contact portion 200c is provided in contact with the epidermis 4. The tape contact portion 202c comes into contact with the adhesive tape 91c on the epidermis 4 side. The tape contact portion 202c comes into contact with the electrode contact portion 210c on the opposite side of the epidermis 4. In other words, the electrode 20c is sandwiched between the adhesive tape 91c and the connection portion 21c in the tape contact portion 202c. Also, in other words, the electrode 20c overlaps the adhesive tape 91c and the connection portion 21c at the tape contact portion 202c. Because the tape contact portion 202c is provided on the adhesive tape 91c, the height from the epidermis 4 is higher than that from the skin contact portion 200c.

The connection portion 201c connects the skin contact portion 200c and the tape contact portion 202c. The connection portion 201c has a bent plate-like shape to connect the skin contact portion 200c and the tape contact portion 202c having different heights from the epidermis 4.

The adhesive tape 91c is attached to the epidermis 4 and provided between the epidermis 4 and the tape contact portion 202c. Thereby, the adhesive tape 91c insulates the electrodes 20c and the epidermis 4 at the tape contact portion 202c.

It is only necessary for the material of the adhesive tape 91c capable of being attached to the epidermis 4 for a long period of time with adhesion to be a material that does not easily have an influence of inflammation or the like on the epidermis 4 like the adhesive portion 22 (FIG. 1). The adhesive tape 91c preferably has breathability. The adhesive tape 91c is a surgical tape for medical use as an example.

The connection portion 21c electrically connects the electrode 20c and the measurement portion 3c. The connection portion 21c is molded into a thin film shape. The connection portion 21c includes an electrode contact portion 210c, a connection portion 211c, and a connector contact portion 212c. The electrode contact portion 210c comes into contact with the surface of the electrode 20c at the tape contact portion 202c. The connector contact portion 212c comes into contact with a connector provided in the measurement portion 3c. The electrode contact portion 210c and the connector contact portion 212c have a thin plate-like shape.

The connection portion 211c connects the electrode contact portion 210c and the connector contact portion 212c. The connection portion 211c has a bent plate-like shape to connect the electrode contact portion 210c and the connector contact portion 212c having different heights from the epidermis 4. Also, when the height of the electrode contact portion 210c from the epidermis 4 is the same as the height of the connector contact portion 212c from the epidermis 4, the connection portion 211c may have a plate-like shape that is not bent.

The measurement portion 3c has a configuration similar to that of the measurement portion 3 (FIG. 3) except for a portion where a connector is provided. In the measurement portion 3c, a connector is provided on the opposite side of the epidermis 4.

The double-sided tape 92c has adhesiveness on both sides. The double-sided tape 92c is attached to the measurement portion 3c on one surface. The double-sided tape 92c is attached to the epidermis 4 on the other side. It is only necessary for the material of at least a surface of the double-sided tape 92c attached to the epidermis 4 within the surface of the double-sided tape 92c to be a material that is able to be attached to the epidermis 4 for a long period of time with adhesion and that does not easily have an influence of inflammation or the like on the epidermis 4 like the adhesive portion 22 (FIG. 1).

Although the adhesive portion 22 shown in FIG. 2 is omitted in the cross-sectional view shown in FIG. 9, a highly breathable adhesive member is attached to the epidermis 4 as well as the electrode contact portion 210c within the connection portion 21c.

In the cross-sectional view shown in FIG. 9, a breathable region R5 indicates a part of the surface of the electrode 20c that is not in contact with the electrode contact portion 210c and is in contact with the epidermis 4. A breathable region R6 indicates a part of the surface of the electrode 20c that is not in contact with the electrode contact portion 210c and the epidermis 4. A non-breathable region R7 indicates a part of the surface of the electrode 20c that is in contact with the adhesive tape 91c. The non-breathable region R7 corresponds to the tape contact portion 202c overlapping the connection portion 21c within the portion of the electrode 20c.

The electrode 20c has breathability. On the other hand, the connection portion 21c does not have breathability. Thus, the electrode portion 2c has breathability in the breathable region R5 and the breathable region R6, but the electrode portion 2 does not have breathability in the non-breathable region R7.

In the skin resistance measurement device 1c, the electrode portion 2c and the epidermis 4 are electrically insulated by the adhesive tape 91c in the non-breathable region R7. Because the electrode portion 2c and the epidermis 4 are electrically insulated, the skin resistance measurement device 1c does not measure the skin resistance of the epidermis 4 in the non-breathable region R7 that does not have breathability.

In the skin resistance measurement device 1c, the electrode portion 2c further includes a skin non-contact portion (in this modified example, the tape contact portion 202c) that is not in contact with the epidermis 4. The skin non-contact portion (in this modified example, the tape contact portion 202c) and the connection portion 21c have a region that overlaps each other (in this modified example, the non-breathable region R7). The skin resistance measurement device 1c includes an insulation portion (in this modified example, the adhesive tape 91c). The insulation portion (in this modified example, the adhesive tape 91c) electrically insulates the skin non-contact portion (in this modified example, the tape contact portion 202c) and the epidermis 4 in the region (in this modified example, the non-breathable region R7).

With this configuration, the skin resistance measurement device 1c can reduce an influence of skin resistance on the measurement result in a region where drying of the epidermis 4 is not naturally performed (in this modified example, the non-breathable region R7).

### [Pattern in which DC voltage is applied]

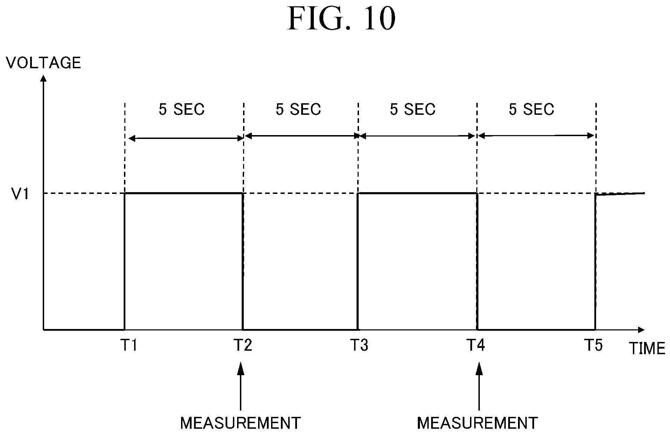

Next, a pattern of a DC voltage applied between the electrode 20-1 and the electrode 20-2 will be described with reference to FIG. 10. FIG. 10 is a diagram showing an example of a pattern in which a DC voltage is applied according to the present embodiment.

The measurement portion 3 applies a DC voltage for a prescribed period of time before measurement and stops the application of the DC voltage for a prescribed period of time when the measurement is performed. Subsequently, an operation in which the measurement portion 3 applies a DC voltage for the prescribed period of time and stops the application of the DC voltage for the prescribed period of time when the measurement is performed is iterated.

In the example shown in FIG. 10, the measurement portion 3 starts the application of a DC voltage having a value V1 between the electrode 20-1 and the electrode 20-2 at time T1. When the DC voltage having the value V1 is applied for 5 sec from time T1 to time T2, the measurement portion 3 measures skin resistance at time T2. The measurement portion 3 stops the application of the DC voltage immediately after performing the measurement at time T2. When 5 sec from time T2 to time T3 have elapsed, the measurement portion 3 starts the application of the DC voltage at time T3. When the DC voltage having the value V1 is applied for 5 sec from time T3 to time T4, the measurement portion 3 measures skin resistance at time T4. The measurement portion 3 stops the application of the DC voltage immediately after performing the measurement at time T4. When 5 sec from time T4 to time T5 have elapsed, the measurement portion 3 starts the application of a DC voltage at time T5. The measurement portion 3 subsequently iterates a similar operation.

The measurement portion 3 does not apply a voltage all the time, but applies a voltage for a period necessary for measurement. Thus, in the skin resistance measurement device 1, the power consumption of the battery 33 can be reduced as compared to the case where a voltage is applied all the time. Also, in the skin resistance measurement device 1, an influence of voltage application on the epidermis 4 can be suppressed as compared with the case where a voltage is applied all the time.

The reason for performing the measurement after applying a DC voltage for a certain period of time is as follows. When the skin is considered as an electric circuit equivalently, there are a resistance component and a capacitance component in the resistance of the skin as described above. Immediately after a voltage is applied to the skin, an electric current flows to charge the capacitance component. Thus, when the resistance value is calculated by dividing the applied voltage by an electric current value, an apparent resistance value decreases and a correct resistance value cannot be measured. In order to avoid this, an accurate resistance value can be measured by applying a voltage during a period when the capacitance component is sufficiently charged and then performing measurement.

Also, due to a cause of body movement or the like, the electrode 20 and the connection portion 21 may primarily cause a contact failure. The resistance value measured in this case becomes infinite because no electric current flows even if a voltage is applied. On the other hand, it is originally necessary to measure the resistance value when 5 sec have elapsed from the voltage application, but it is also assumed that data will be measured immediately after the contact failure is restored. In this case, because an electric current for charging the capacitance component flows, the resistance value smaller than the accurate resistance value is measured.

For the purpose of removing such erroneous data, the measurement portion 3 may include a detection circuit. The detection circuit detects whether or not the electrode 20 and the connection portion 21 have been normally connected. On the basis of a detection result of the detection circuit, the data measured when the contact failure occurs or immediately after the contact failure may be excluded from the measurement data.

Although a sequence for measuring the resistance value after applying a voltage for a certain period of time is shown in FIG. 10, the present invention is not limited thereto. The resistance value may be measured at regular time intervals while a voltage is continuously applied without providing a period of time to pause voltage application. In this case, there is an advantage that the resistance value can be measured at short intervals as compared to the case where a pause period of time is set for the voltage application.

Hereinafter, results of measuring skin resistance under various conditions using the skin resistance measurement device 1 according to the present embodiment will be described. In each embodiment example described below, the electrode 20 has a nanomesh structure in which gold is deposited on the nanofiber network as described above.

### [First embodiment example]

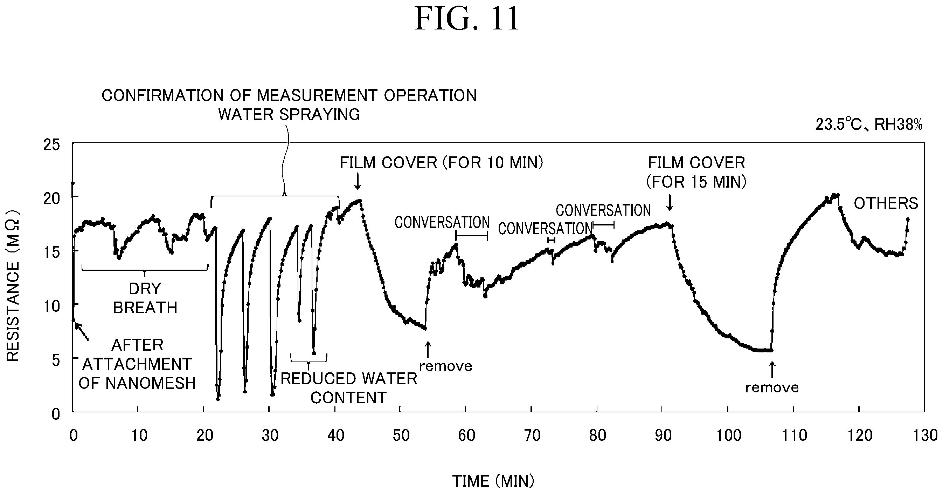

FIG. 11 is a diagram showing an example of a result of measuring a change over time in skin resistance according to the present embodiment example. In the example shown in FIG. 11, the electrodes 20 provided in the skin resistance measurement device 1 are attached to the epidermis 4 of a measurement target person at the time of 0 min. The measurement was performed continuously for about 120 min. At the time of measurement, a room temperature is 23.5 degrees and humidity is 38%.

After the electrodes 20 were attached to the epidermis 4, the electrodes 20 were dried for about 20 min. In the process of drying, the breath was blown to the electrodes 20. When the drying was completed, the measurement operation was confirmed for about 20 min. In a process of confirming the measurement operation, the electrodes 20 were sprayed with a very small water content at prescribed intervals. Immediately after the electrodes 20 were sprayed with water, the measured skin resistance decreased and the skin resistance increased as it dried. When the water content with which the electrodes 20 were sprayed was reduced, the skin resistance did not decrease as compared with the case where the water content was not reduced.

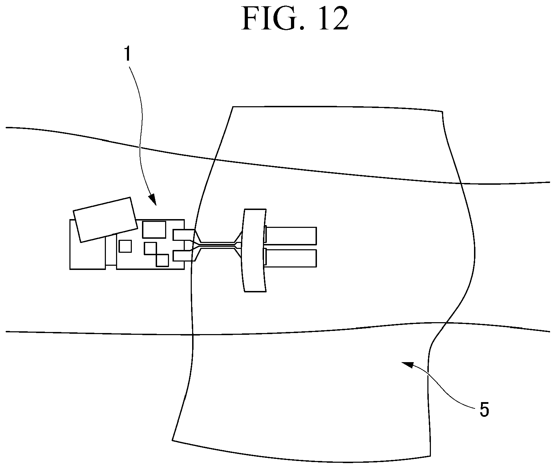

Next, when about 45 min elapsed after the start of measurement, the electrodes 20 were covered with the film cover 5. In FIG. 12, the appearance of the skin resistance measurement device 1 attached to the epidermis 4 in a state in which the electrodes 20 were covered with the film cover 5 is shown. In the present embodiment example, the epidermis 4 was the forearm of the measurement target person. The film cover 5 was a polyethylene film with a thickness of 50 µm as an example. When the electrodes 20 were covered with the film cover 5, the epidermis 4 was also covered with the film cover 5 and water transpiration from the skin surface was blocked. As a result, skin resistance decreased immediately after the electrodes 20 were covered with the film cover 5. When 10 min elapsed after the electrodes 20 were covered with the film cover 5, the film cover 5 was removed. Because the film cover 5 was removed, natural water transpiration from the skin resumed and skin resistance began to increase.

Subsequently, the measurement target persons had a conversation. Skin resistance tended to decrease during the conversation. When about 90 min elapsed from the start of measurement, the electrodes 20 were again covered with the film cover 5. When 15 min elapsed after the electrodes 20 were covered with the film cover 5, the film cover 5 was removed. Because the film cover 5 was removed, skin resistance began to increase.

Next, a case where measurement of the skin resistance measurement device 1 can be performed continuously for a long period of time and a measurement result is reproducible will be described.

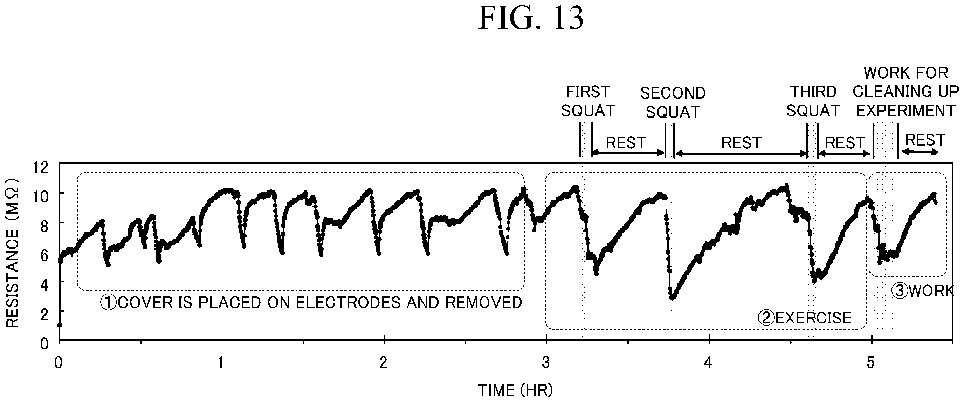

FIG. 13 is a diagram showing an example of a result of measuring a change over time in skin resistance according to the present embodiment example. In FIG. 13, a first half (a period of about 3 hours from the start of measurement) shows a change in skin resistance in a non-exercise state and a second half (a period of about 3 to 5 hours from the start of measurement) shows a change in skin resistance when exercise was done. The non-exercise state is a state in which exercise was not done. The exercise was doing 30 squats as an example. The measurement method was common between the first half and the second half and the skin resistance measurement device 1 was attached to the skin. The measurement of the first half and the measurement of the second half were performed continuously and the total measurement time was 5 hours or more. In a period of about 3 hours from the start of the measurement result corresponding to the first half, an operation in which the electrodes 20 were covered with the film cover 5 and removed was iterated.

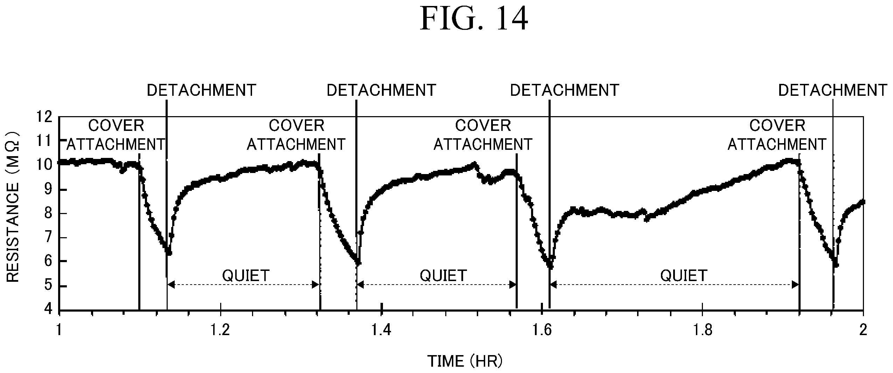

A change over time in skin resistance obtained by extracting and enlarging a measurement result for a period of 1 to 2 hours from the start of measurement among the measurement results is shown in FIG. 14. In FIG. 14, covering the electrodes 20 with the film cover 5 is shown as "cover attachment," and removing the film cover 5 is shown as "detachment."

During this period, a skin resistance value when about 5 min elapsed after the electrodes 20 were covered with the film cover 5 and a skin resistance value increased with water transpiration from the skin surface after the film cover 5 was removed were substantially uniform and the skin resistance value was stable between minimum and maximum values.

Description continues with reference back to FIG. 13. During the period of 3 to 5 hours from the start of the measurement, an operation in which the measurement target person had a rest after exercising with squats was iterated. During the exercise, sweating reduced the skin resistance value. During the rest, the skin resistance value increased due to the transpiration of sweat. During the period in which the exercise and rest were iterated, the skin resistance value was substantially uniform between the value immediately after exercise and the value after rest and the skin resistance value was stable between minimum and maximum values.

Also, after 5 hours from the start of the measurement, the measurement target person had a rest after the work for cleaning up the experiment related to the measurement.

From the measurement results shown in FIGS. 13 and 14, the possibility of continuous measurement using the skin resistance measurement device 1 for a long period of time of 5 hours or more and that reproducibility were confirmed.

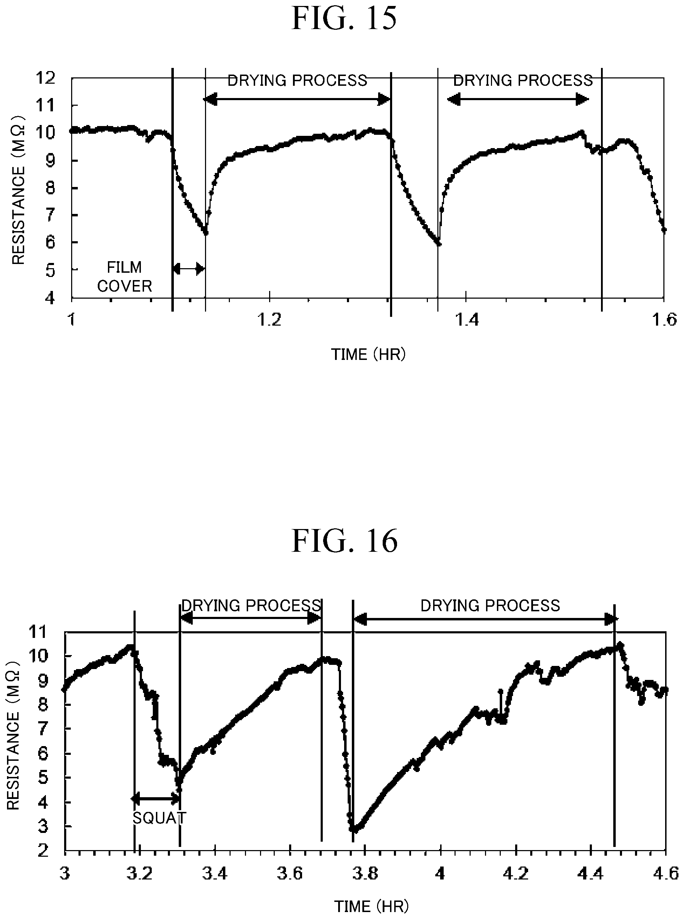

Next, processes of decreasing and increasing skin resistance by non-exercise (film attachment and detachment) and exercise (squats) are compared with reference to FIGS. 15 and 16.

FIG. 15 is a diagram showing an example of a change in skin resistance that occurs when the film is attached and detached according to the present embodiment example. The change in skin resistance is considered to be a phenomenon relating to water transpiration from the skin and can be visualized in more detail in the skin resistance measurement device 1 than in the conventional technology. FIG. 15 is a diagram obtained by extracting and enlarging a measurement result for a period of 1 to 1.6 hours from the start of measurement among the measurement results shown in FIG. 13. FIG. 16 is a diagram showing an example of a sweating and drying process due to exercise according to the present embodiment example. FIG. 16 is a diagram obtained by extracting measurement results for a period of 3 to 4.6 hours from the start of measurement among the measurement results shown in FIG. 13.

When FIGS. 15 and 16 are compared, a method in which the skin resistance decreases is different between a case where the electrodes 20 were covered with the film cover 5 and a case where sweat was generated due to exercise. Also, a method in which the skin resistance increases is different between a drying process due to water transpiration from the skin after removing the film cover 5 and a drying process after sweating by exercise. In the drying process due to water transpiration from the skin, the method in which the skin resistance increases is a method in which an increasing rate in the skin resistance decreases with time. On the other hand, in the drying process after sweating by exercise, the skin resistance increases in proportion to time.

Thus, the skin resistance measurement device 1 can measure skin resistance at high accuracy to a degree that skin resistance change mechanisms are identified in a case where a drying process occurs due to water transpiration from the skin after the epidermis 4 was covered with the film cover 5 and then removed and a case where a drying process occurs after sweating due to exercise.

### [Second embodiment example]

When an adhesive plaster or polyurethane film was applied to the epidermis, an influence on skin resistance was assessed.

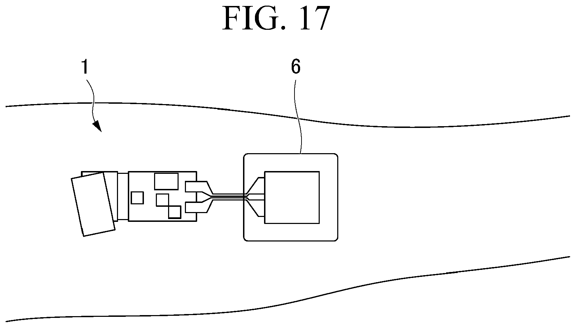

FIG. 17 shows the appearance of the skin resistance measurement device 1 attached to the epidermis 4 in a state in which the electrodes 20 were covered with the adhesive plaster 6. The adhesive plaster was attached to the epidermis 4 at a position where the electrodes 20 were covered. The adhesive plaster had a substantially square shape with one side of about 35 mm. Therefore, the size of the adhesive plaster was smaller than the size of the film cover 5 used in the above-described first embodiment example.

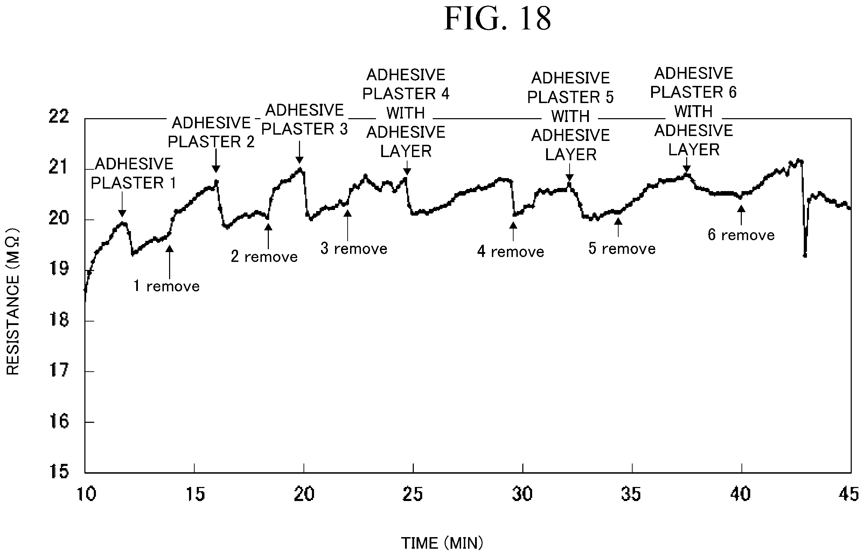

FIG. 18 is a diagram showing an example of a result of measuring a change over time in skin resistance when the adhesive plaster 6 was attached to the epidermis 4 according to the present embodiment example. In FIG. 18, arrows from "adhesive plaster 1" to "adhesive plaster 3" indicate measurement results immediately after adhesive plasters without adhesive layers were attached and arrows from "adhesive plaster 4 with adhesive layer" to "adhesive plaster 6 with adhesive layer" indicate measurement results immediately after adhesive plasters with adhesive layers were attached. In the measurement, an operation in which the adhesive plaster 6 was attached to the epidermis 4 and removed was iterated. After the adhesive plaster 6 was attached, the adhesive plaster 6 was removed when the skin resistance decreased and stabilized. After the adhesive plaster 6 was removed, the adhesive plaster 6 was attached again when the skin resistance increased and stabilized. From the measurement results shown in FIG. 18, it can be seen that skin resistance reacted sensitively even if an adhesive plaster having a smaller area than the film cover 5 was placed. The adhesive plaster and the film cover are examples of materials attached to the skin, and the skin resistance measurement device 1 can assess influences of a wide range of materials on the skin. Other examples of materials whose influences on the skin can be assessed using the skin resistance measurement device 1 include gauze or cloth, clothing that covers the skin on a daily basis, agents for protecting and moisturizing the skin, and the like. Regarding the agent for moisturizing, an influence on the skin when a commercially available moisturizer or the like is applied directly on the electrode 20 attached to the skin has already been assessed by the skin resistance measurement device 1.

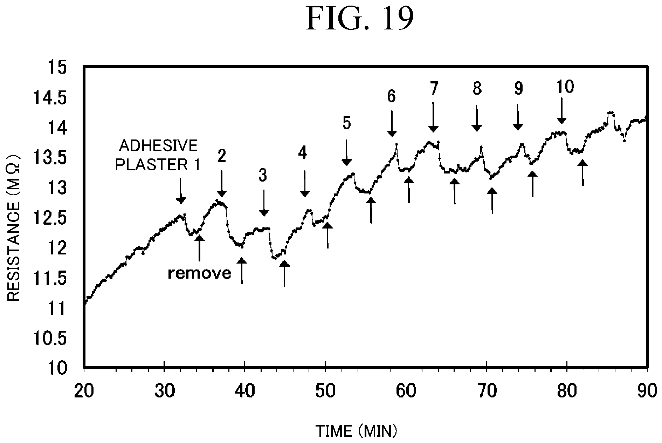

FIG. 19 is a diagram showing an example of a measurement result for verifying the reproducibility of an influence on skin resistance when the adhesive plaster 6 was attached to the epidermis 4 according to the present embodiment example. In the measurement results shown in FIG. 19, an operation of attaching the adhesive plaster 6 to cover the electrodes 20 and removing the adhesive plaster 6 was iteratively performed after 32 min in a period of 20 to 90 min after the skin resistance measurement device 1 was attached to the epidermis 4. The operation was iterated 10 times, but it can be seen that the influence on skin resistance was iteratively reproduced when the adhesive plaster 6 was attached to the epidermis 4 from a change over time in skin resistance shown in FIG. 19.

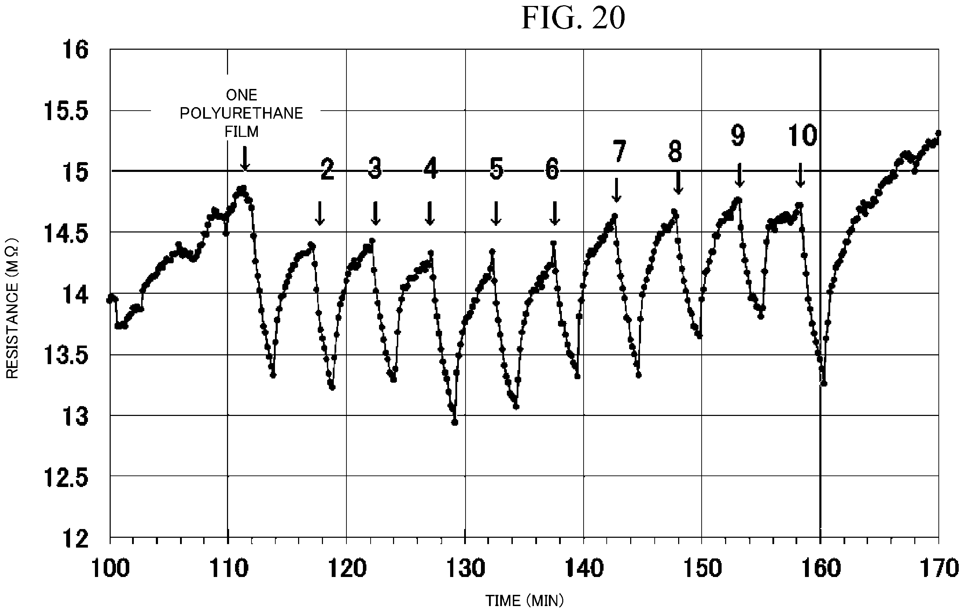

Here, a measurement result of performing measurement using a polyurethane film 7 instead of the adhesive plaster 6 is shown to verify the reproducibility of the influence on skin resistance. FIG. 20 is a diagram showing an example of a measurement result for verifying the reproducibility of the influence on skin resistance when the polyurethane film 7 was attached to the epidermis 4. In the measurement results shown in FIG. 19, an operation of attaching the polyurethane film 7 to cover the electrodes 20 and removing the polyurethane film 7 was iteratively performed after 112 min in a period of 100 to 170 min after the skin resistance measurement device 1 was attached to the epidermis 4. The operation was iterated 10 times, but it can be seen that the influence on skin resistance was iteratively reproduced when the polyurethane film 7 was attached to the epidermis 4 from a change over time in skin resistance shown in FIG. 19.

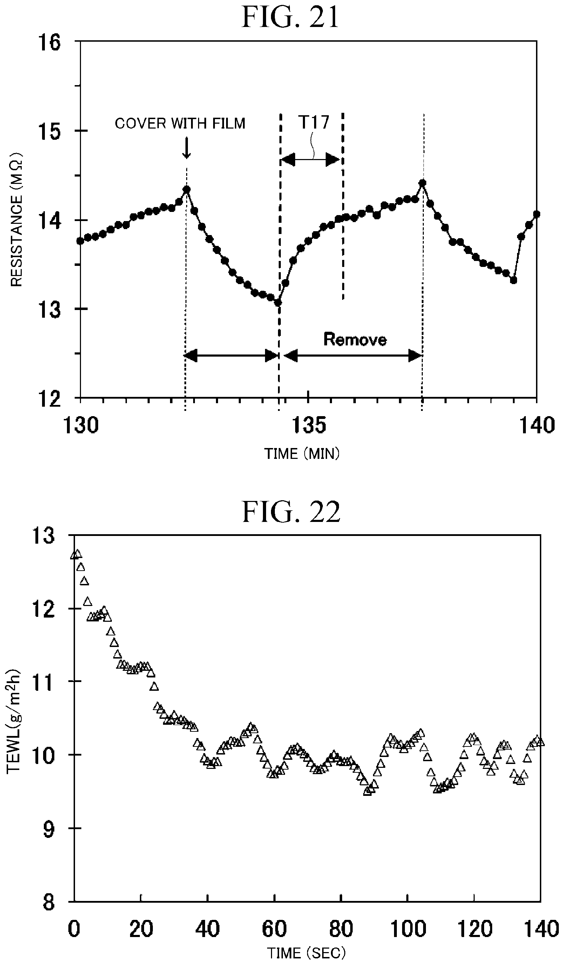

Next, a result of measuring skin resistance using the skin resistance measurement device 1 is compared with a measurement result other than that of skin resistance using a skin measurement instrument to be compared with reference to FIGS. 21 to 25. In the present embodiment example, transepidermal water loss (TEWL) was measured as a measurement result other than that of skin resistance.

FIG. 21 is a diagram showing an example of a result of measuring skin resistance when the film cover 5 was attached to the epidermis 4 to cover the electrodes 20 and removed according to the present embodiment example. In FIG. 21, a measurement result for a period of 130 to 140 min after the start of measurement is shown. When the film cover 5 was removed as described above, drying due to transpiration began. In the present embodiment example, in the period T17 shown in FIG. 21, a result of measuring skin resistance using the skin resistance measurement device 1 was compared with a measurement result of TEWL using the skin measurement instrument to be compared. In the period T17, the length of the period T17 was about 140 sec in correspondence with the drying process due to transpiration after the film cover 5 was removed.

FIG. 22 is a diagram showing an example of a TEWL measurement result using a skin measurement instrument to be compared according to the present embodiment example. The measurement result was a measurement result of a drying process due to transpiration for 140 sec after the film cover 5 was removed. Also, measurement could not be performed with the skin measurement instrument to be compared when the film cover 5 was attached.

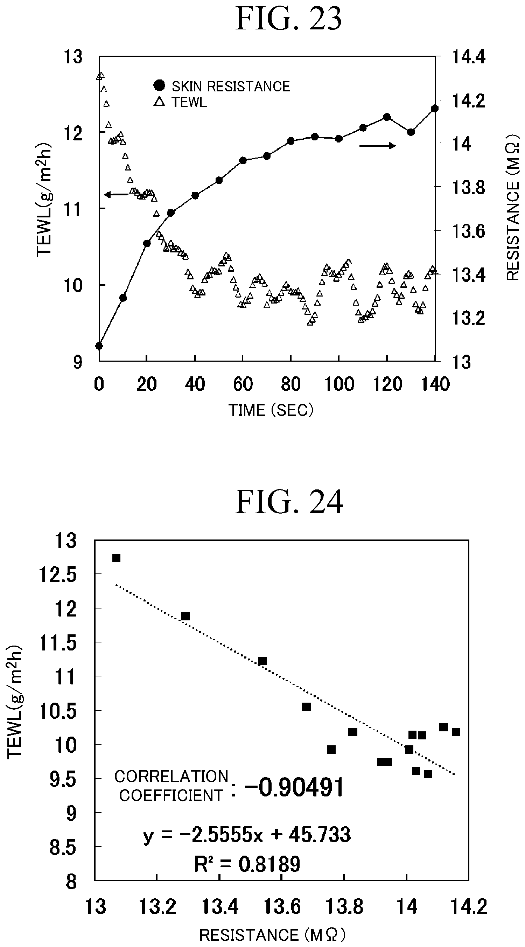

FIG. 23 is a diagram showing an example in which the result of measuring skin resistance using the skin resistance measurement device 1 according to the present embodiment example is compared with the result of measuring TEWL using the skin measurement instrument to be compared. According to the measurement results shown in FIG. 23, it can be seen that there was a strong correlation between skin resistance and TEWL in the drying process due to transpiration. A result of calculating a correlation coefficient for a correlation is shown in FIG. 24. The correlation coefficient was -0.90491.

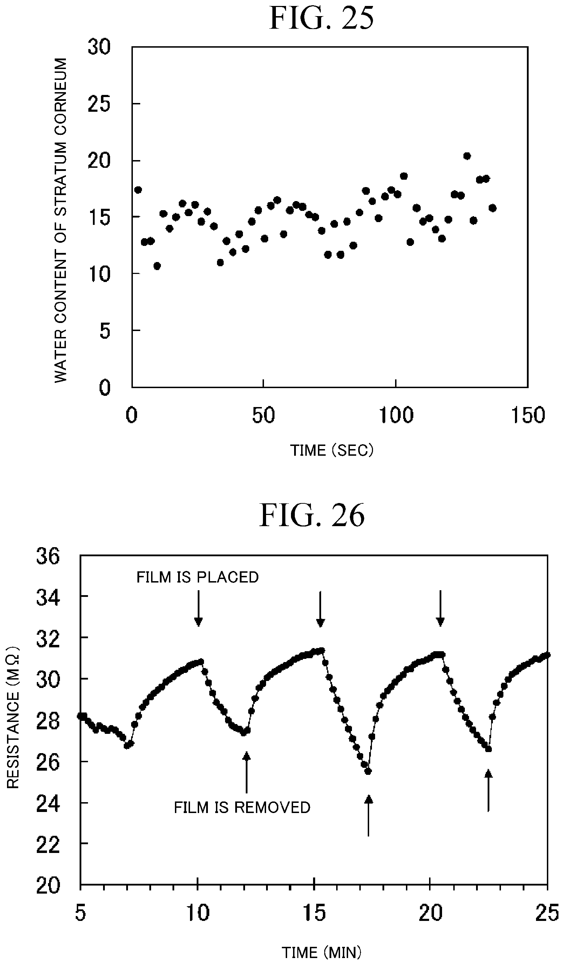

For comparison, a result of measuring a water content (arbitrary unit) of the stratum corneum using the skin measurement instrument to be compared after removing the film cover 5 is shown in FIG. 25. As can be seen from FIG. 25, while transpiration was occurring in the epidermis after removing the film cover 5, no significant trend was observed regarding a change over time in the water content of the stratum corneum. Therefore, it can be seen that there was no correlation between skin resistance and the water content of the stratum corneum in the drying process due to transpiration.

### [Third embodiment example]

Next, an example in which a difference in the material of the film cover with which the electrodes 20 are covered or a difference in the constitution of the measurement target person has an influence on skin resistance will be described.

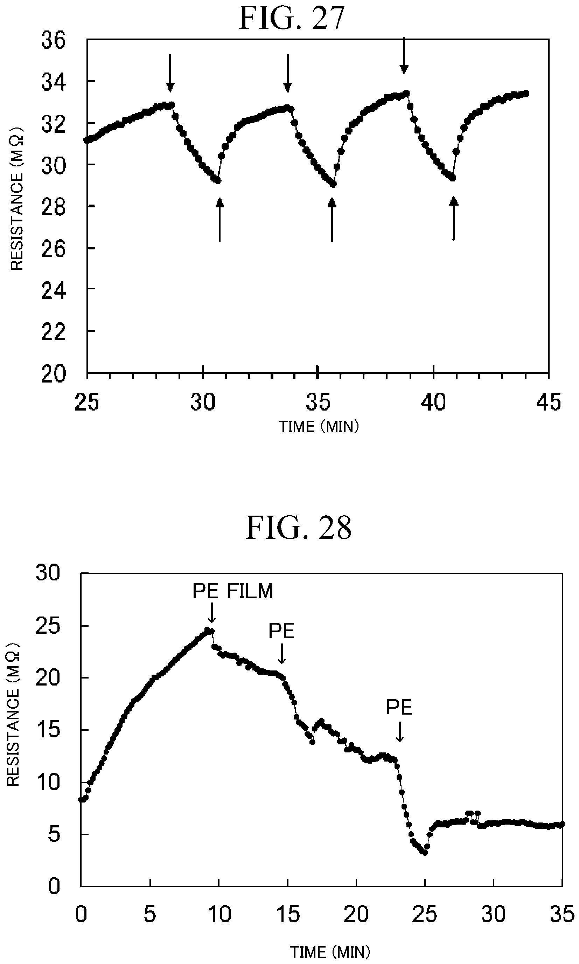

FIG. 26 shows a result of measuring skin resistance when polyethylene (PE) having a thickness of 50 µm was used as a film cover with which the electrodes 20 were covered. FIG. 27 shows a result of measuring skin resistance when polyurethane (PU) having a thickness of 75 µm was used as the film cover with which the electrodes 20 were covered. In both the measurement results shown in FIGS. 26 and 27, the measurement target person is a man in his 30s.

In any of the measurement results shown in FIGS. 26 and 27, an operation in which the film cover was removed and dried for 3 min after the electrodes 20 were covered with the film cover and left for 2 min was iteratively performed. In any of the measurement results shown in FIGS. 26 and 27, skin resistance after 2 min from covering with the film cover and the skin resistance after 3 min from the removal of the film cover were each substantially constant between iterated operations.

In the measurement results shown in FIGS. 26 and 27, there is a possibility that a stimulation received by the skin will differ according to a material of the film cover and a process of water transpiration from the skin will be different.

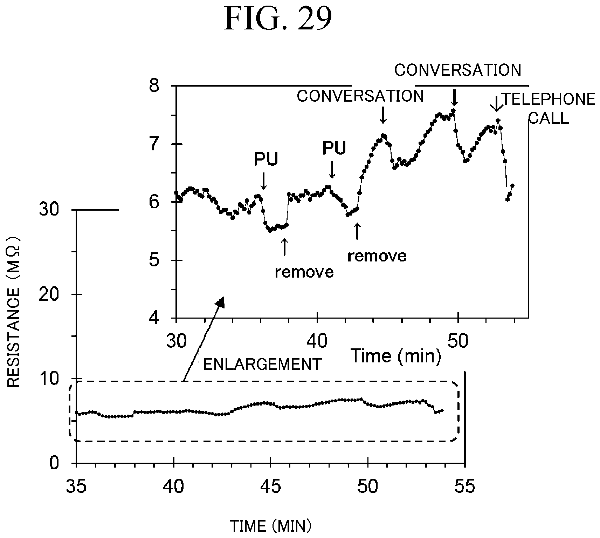

FIG. 28 shows a measurement result when the measurement target person was a man in his 60s and PE was used as the film cover. FIG. 29 shows a measurement result when the measurement target person was the man and PU was used as a film cover.

In the measurement results shown in FIG. 28, skin resistance tended to gradually decrease as an operation of covering the electrodes 20 with a film cover and removing it was iterated. After 25 min from the start of measurement, skin resistance did not increase again. The reason is that the measurement target person has a constitution that is prone to perspiration or has a skin disease.

In FIG. 29, the measurement target person was talking or making a telephone call along with the operation of covering the electrodes 20 with a film cover and removing it. In the measurement results of FIG. 29, it can be seen that these conversations or actions such as telephone calls affected skin resistance.

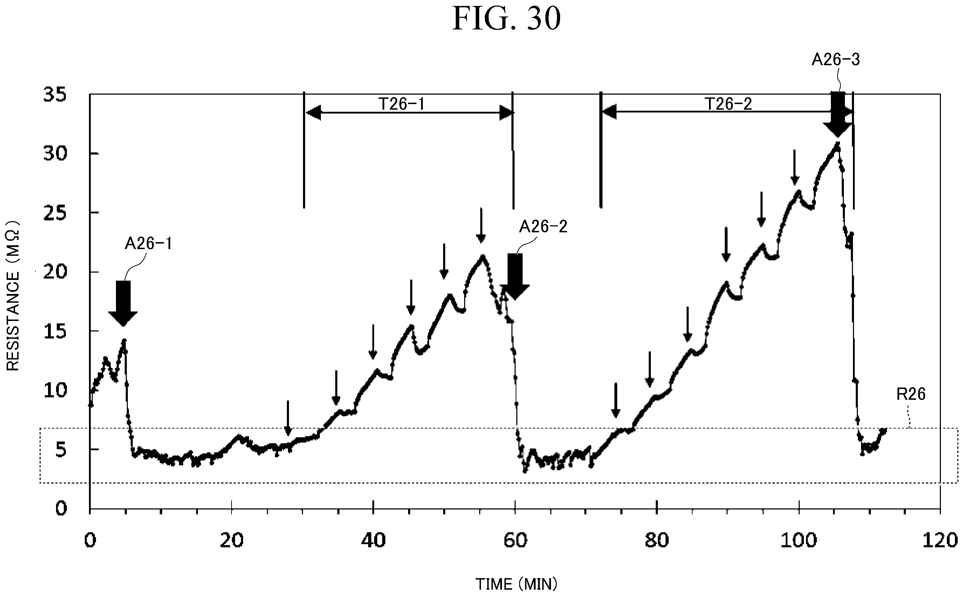

FIG. 30 shows a measurement result when the measurement target person was a woman in her 40s and PE was used as the film cover. The measurement target person was often tense during the measurement and the skin resistance decreased sharply in the tense state. In FIG. 30, a period when tension is estimated to have begun is indicated by an arrow A26-1, an arrow A26-2, and an arrow A26-3.

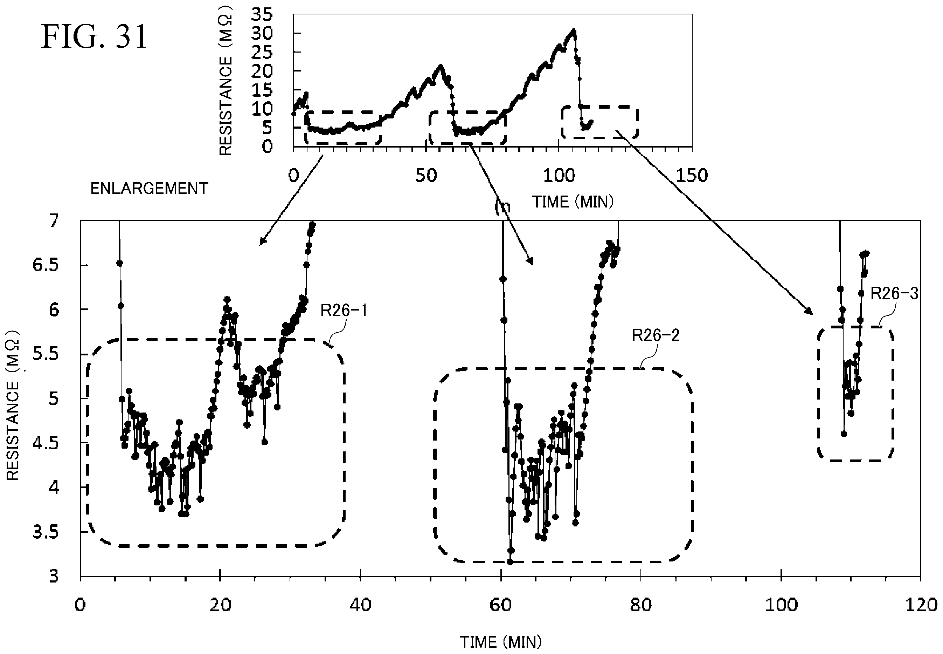

A result of enlarging a measurement result included in a range R26 shown in FIG. 30 in a direction of the skin resistance value (i.e., in a direction of the vertical axis) is shown in FIG. 31. In FIG. 31, the range R26 shown in FIG. 30 is divided into three ranges, i.e., a range R26-1, a range R26-2, and a range R26-3. From FIG. 31, it can be seen that the skin resistance vibrated violently even though an operation of covering the electrodes 20 with a film cover and removing it was not performed in the three ranges, i.e., the range R26-1, the range R26-2, and the range R26-3.

Also, in periods T26-1 and T26-2, the measurement target persons tried to relieve tension by closing their eyes and calming their feelings. In the periods T26-1 and T26-2, a tendency of skin resistance to vibrate violently observed in the tension state was not observed and the skin resistance was stable and gradually increased. This change is thought to be due to the change from a state in which the sympathetic nervous system is dominant to the parasympathetic nervous system when the eyes were closed and the gradual increase in skin resistance reflecting a result thereof. Thus, the baseline of skin resistance gradually increased, but a study of iteratively performing an operation of covering the electrodes 20 with a film cover and removing it at the same time in the periods T26-1 and T26-2 was conducted.

From the measurement results shown in FIGS. 30 and 31, it can be seen that the tension and emotional state of the measurement target person were reflected in the skin resistance. Thus, it can be said that the skin resistance measurement device 1 according to the present embodiment showed a possibility of estimation of a state associated with individual tension and emotions from fine changes in skin resistance just by simply attaching it to the skin of the forearm or the like.

### [Fourth embodiment example]

In order to confirm an effect that the electrode 20 has a structure in which drying of the epidermis 4 is naturally performed, measurement was performed by removing the electrode 20 from the skin resistance measurement device 1 as a comparative example.

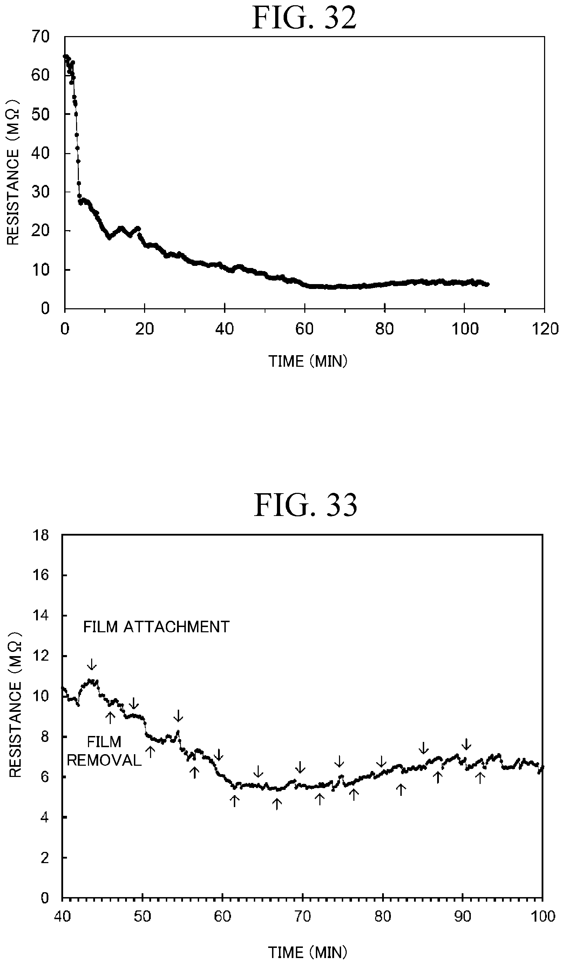

FIG. 32 is a diagram showing an example of a result of measuring skin resistance according to a comparative example of the present embodiment example. The measurement result shown in FIG. 32 is a result of performing measurement by removing the electrode 20 from the skin resistance measurement device 1 and causing the connector of the measurement portion 3 to directly come into contact with the epidermis 4. As shown in FIG. 32, the value of skin resistance decreases over time and does not return to its original value once it has decreased. Because the electrode 20 was removed from the skin resistance measurement device 1 and drying of the epidermis 4 was not performed naturally, it is considered that moisture such as sweat accumulated over time.

Next, the influence on skin resistance when an operation of attaching the film cover to the epidermis 4 and removing it was iterated are compared between a case where the skin resistance measurement device 1 had the electrodes 20 and a case where the electrode 20 was removed from the skin resistance measurement device 1.

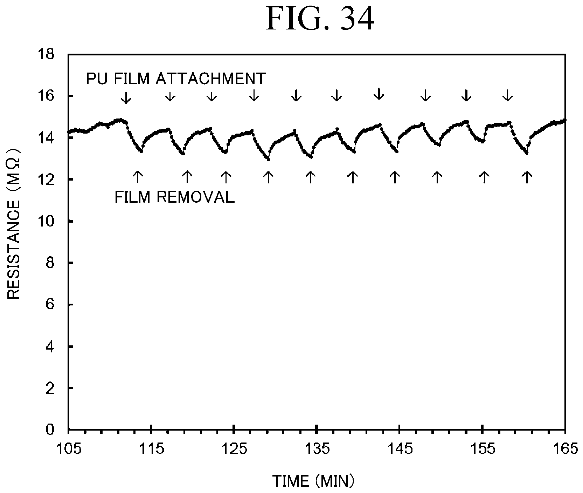

FIG. 33 is a diagram showing an example of a result of measuring skin resistance according to a comparative example of the present embodiment example. The measurement result shown in FIG. 33 is a result of performing measurement by removing the electrodes 20 from the skin resistance measurement device 1 and causing the connector of the measurement portion 3 to directly come into contact with the epidermis 4. When the film cover was attached to the epidermis 4 in a state in which the electrodes 20 were removed from the skin resistance measurement device 1, the film cover was attached to cover the connector of the measurement portion 3. FIG. 34 is a diagram showing an example of a result of measuring skin resistance according to the present embodiment example. The measurement result shown in FIG. 34 is a measurement result of a case where the skin resistance measurement device 1 had the electrodes 20.

In FIGS. 33 and 34, the time when the film cover was attached to the epidermis 4 is indicated by a downward arrow and the time when the film cover was removed is indicated by an upward arrow. From FIG. 34, when the skin resistance measurement device 1 had the electrodes 20 having the structure in which drying of the epidermis 4 was naturally performed, an influence of the film cover on skin resistance could be accurately measured. On the other hand, from FIG. 33, it can be seen that the influence could not be entirely accurately measured in comparison with the measurement result shown in FIG. 34 when the connector of the measurement portion 3 was brought into direct contact with the epidermis 4 and measurement was performed. Also, as described in FIG. 32, when the connector of the measurement portion 3 was brought into direct contact with the epidermis 4 and measurement was performed, moisture such as sweat was considered to be accumulated over time and the value of skin resistance tended to decrease and was not as stable as the measurement result shown in FIG. 34.

According to the measurement results of the above-described embodiment examples, it can be seen that the skin resistance measurement device 1 can measure skin resistance with such accuracy that a state in which the measurement target person is talking, an exercising state, a tense state, or the like can be distinguished according to skin resistance. That is, the skin resistance measurement device 1 can be fully applied to determine the behavior and/or emotion of the measurement target person.

The behavior/emotion determination method using a measurement result of the skin resistance measurement device 1 includes, for example, an attachment step, a measurement step, and a determination step. In the attachment step, the electrodes 20 are attached to the epidermis of the measurement target person with an adhesive tape or the like and the skin resistance measurement device 1 is attached to the measurement target person. In the measurement step, the skin resistance between the electrodes 20 is measured by applying a DC voltage between the electrodes 20. In the determination step, the behavior and/or emotion of the measurement target person are/is determined on the basis of the skin resistance measured in the measurement step. A pattern of a change over time in skin resistance is pre-associated with the content of behavior or emotion. Information indicating this association is referred to as pattern information.

As an example, the determination step is executed by a determination device separate from the skin resistance measurement device 1. In this case, the skin resistance measurement device 1 outputs the skin resistance value measured in the measurement step to the determination device. The determination device stores the pattern information and determines the behavior and/or emotion of the measurement target person on the basis of the information and a skin resistance value measured by the skin resistance measurement device 1. Examples of the determination device are a smartphone, a personal computer (PC), a server, and the like.

When the skin resistance value is output, the skin resistance measurement device 1 and the determination device perform wireless or wired communication. In the skin resistance measurement device 1, the measurement portion 3 includes a communication module for communicating with the determination device.

Also, the determination step may be performed by the skin resistance measurement device 1. In this case, the skin resistance measurement device 1 stores a program for executing the determination step and the pattern information in the memory 32.

Also, the responsiveness of the skin to the external environment and the change in life behavior of the measurement target person may be assessed using the measurement result of the skin resistance measurement device 1. A skin responsiveness assessment method for use in the skin resistance measurement device 1 includes, for example, an attachment step, a measurement step, and an assessment step. The attachment step and the measurement step are similar to the attachment step and the measurement step provided in the above-described behavior/emotion determination method. In the assessment step, the responsiveness of the skin to the external environment and a change in life behavior of the measurement target person is assessed on the basis of the skin resistance measured in the measurement step.

The external environment includes temperature and humidity, climate, measurement locations, measurement conditions, and the like.

A change in life behavior is content relating to various types of behavior performed in daily life, and broadly includes behavior such as daily activities including a conversation, a telephone call, exercise, use of beauty and skin care products, application of external chemicals, use of skin patch sheets, putting on and taking off clothes, sleeping, and waking up.

The responsiveness of the skin defined here is assessed from the change in skin resistance. A state of the change in the skin resistance is indicated by an increase or decrease in the skin resistance, a rate of change over time, and the like.

Specific examples of the skin responsiveness assessment will be described.

First, the assessment of the responsiveness of the skin to a change in life behavior will be described with reference to FIGS. 11 and 29. From a measurement result for the period during which the conversation shown in FIG. 11 was being conducted (between 50 min and 90 min) or a measurement result for the period during which the conversation or telephone call shown in FIG. 29 was being performed, it can be seen that skin resistance was temporarily reduced due to a change in behavior such as a conversation.

Next, the responsiveness of the skin to the external environment will be assessed and described. When FIGS. 15 and 16 are compared, a period of time until the resistance value returned to about a value before the electrodes were covered with the film cover in the drying process after the application and removal of the film cover shown in FIG. 15 is longer than a period of time until the resistance value returned to about a value before the squat was performed in the drying process after the squat was performed shown in FIG. 16. Also, it can be seen that the return of the resistance value in the drying process after the squat was performed in the change in skin resistance shown in FIG. 16 is close to a linear change as compared with the return of the resistance value in the drying process after the film cover was removed in the change in skin resistance shown in FIG. 15.

Also, when FIGS. 15 and 18 are compared, the state of a change in skin resistance after applying and removing the film cover shown in FIG. 15 and the state of the change in a resistance value after the application and removal of the adhesive plaster shown in FIG. 18 are different. From the difference between the states of these changes, it can be seen that the skin responsiveness differs depending on the external environment.

As described above, the responsiveness of the skin to the external environment or a change in life behavior can be assessed using the measurement results of the skin resistance measurement device 1.

### [Summary]

As described above, the skin resistance measurement device 1 according to the present embodiment includes the pair of the electrode portions 2 to be provided on the epidermis 4 and the measurement portion 3 configured to measure skin resistance between the pair of the electrode portions 2. The pair of the electrode portions 2 has a structure (in the present embodiment, a nanomesh structure) in which drying of the epidermis 4 is naturally performed.

With this configuration, the skin resistance measurement device 1 according to the present embodiment can perform measurement for a long period of time without blocking natural water transpiration from the skin when the electrodes are directly attached to the skin because the pair of electrode portions to be provided on the epidermis has the structure in which drying of the epidermis is naturally performed when skin resistance is measured by directly attaching a sensor such as the skin resistance measurement device 1 to the skin of the measurement target person.

The skin resistance measurement device 1 according to the present embodiment can be used for a long period of time and can measure various processes relating to a skin state such as water transpiration from the skin or an influence on a change in the external environment over time. In the skin resistance measurement device 1 according to the present embodiment, it is possible to continuously monitor the resistance value of the skin close to the natural state for a long period of time without restricting behavior.

Also, as shown in FIGS. 23 and 24, it can be clarified that the skin resistance value correlates with TEWL (transepidermal water transpiration amount), which is an important index of a skin barrier. By continuously measuring skin resistance over a long period of time, it is possible to clarify a close relationship between daily behavior and a skin state. Because the (genetic) characteristics of an individual are reflected in the relationship between the daily behavior and the skin state, a result of measuring skin resistance can be used to assess life behavior at a general level or an individual level directly linked to skin health or aging.

Also, by analyzing the data measured by the skin resistance measurement device 1 according to the present embodiment in association with other multimodal clinical information, it is expected that biomarkers for diagnosis or monitoring that could only be known invasively until now can be assessed by non-invasive measurement. It is expected to predict the onset of disease and the deterioration of a skin state by assessing the responsiveness of the skin to an environmental change and further propose an individualized skin care method on the basis of a prediction result. Also, data measured by the skin resistance measurement device 1 is considered to be closely related to a genetic factor and a disease state and the data is expected to be utilized for diagnosis or a biomarker.

Also, the skin resistance measurement device 1 according to the present embodiment includes the pair of the electrode portions 2 to be provided on the epidermis 4 and the measurement portion 3 configured to measure skin resistance between the pair of the electrode portions 2. The electrode portion 2 has a skin contact portion (in the present embodiment, the electrode 20) that is in contact with the epidermis 4 and has a structure (in the present embodiment, a nanomesh structure) in which drying of the epidermis 4 is naturally performed and the connection portion 21 configured to electrically connect the electrode portion 2 and the measurement portion 3.

With this configuration, because the skin resistance measurement device 1 according to the present embodiment can absorb the external force by bending the connection portion 21 when an external force is applied to the device, it is less likely to be damaged than when the connection portion 21 is not provided.

Also, the skin resistance measurement device 1 according to the present embodiment includes the pair of the electrode portions 2 to be provided on the epidermis 4 and the measurement portion 3 configured to measure skin resistance between the pair of the electrode portions 2 by applying a DC voltage to the pair of the electrode portions 2. The pair of the electrode portions 2 has a structure (in the present embodiment, a nanomesh structure) in which drying of the epidermis 4 is naturally performed.

With this configuration, the skin resistance measurement device 1 according to the present embodiment can measure the DC resistance value of the skin, unlike the case where an AC voltage is applied.

Although an example in which the electrodes are provided on the epidermis of the forearm of the measurement target person and measurement is performed has been described in the above-described embodiment, the present invention is not limited thereto. The electrodes may be provided on the skin of another part of the body of the measurement target person.

Although an example in which the measurement target of the skin resistance measurement device is a human has been described in the above-described embodiment, the present invention is not limited thereto. The skin resistance measurement device may be attached to the skin of an animal other than humans and used to measure skin resistance.

Although embodiments of the present invention have been described in detail with reference to the drawings, specific configurations are not limited to those described above and various design changes and the like can be made without departing from the scope and spirit of the present invention.

### [Reference Signs List]

1, 1a, 1b, 1c Skin resistance measurement device
2, 2a, 2c Electrode portion
20, 20a, 20b, 20c Electrode
21, 21a, 21b, 21c Connection portion
3, 3c Measurement portion
4 Epidermis

## Claims

1. A skin resistance measurement device comprising:
a pair of electrode portions to be provided on an epidermis; and
a measurement portion configured to measure skin resistance between the pair of electrode portions,
wherein the pair of the electrode portions has a structure in which drying of the epidermis is naturally performed.

2. The skin resistance measurement device according to claim 1, wherein the electrode portion has a fiber network.

3. The skin resistance measurement device according to claim 1, wherein the electrode portion has fine linear members arranged at prescribed intervals.

4. The skin resistance measurement device according to claim 1, wherein the electrode portion has a stripe-shaped opening.

5. The skin resistance measurement device according to claim 1, wherein the electrode portion is a foil-shaped electrode with a crack or a pinhole.

6. The skin resistance measurement device according to claim 1, wherein the electrode portion has a structure that releases moisture absorbed on a surface in contact with the epidermis.

7. A skin resistance measurement device comprising:
a pair of electrode portions to be provided on an epidermis; and
a measurement portion configured to measure skin resistance between the pair of electrode portions,
wherein the electrode portion includes an epidermis contact portion that is in contact with the epidermis and has a structure in which drying of the epidermis is naturally performed and a connection portion configured to electrically connect the electrode portion and the measurement portion.

8. The skin resistance measurement device according to claim 7, wherein the connection portion has breathability.

9. The skin resistance measurement device according to claim 7 or 8, wherein an area of a part in contact with the connection portion is smaller than an area of a part not in contact with the connection portion in a surface of the epidermis contact portion.

10. The skin resistance measurement device according to any one of claims 7 to 9, wherein a conductive member having breathability is inserted between the electrode portion and the connection portion.

11. The skin resistance measurement device according to claim 7,
wherein the electrode portion further includes an epidermis non-contact portion not in contact with the epidermis,
wherein the epidermis non-contact portion and the connection portion have a mutually overlapping region, and
wherein the skin resistance measurement device further comprises an insulation portion configured to electrically insulate the epidermis non-contact portion and the epidermis in the region.

12. The skin resistance measurement device according to any one of claims 7 to 11, further comprising a detection circuit configured to detect whether or not the electrode portion has been connected to the connection portion.

13. A skin resistance measurement device comprising:
a pair of electrode portions to be provided on an epidermis; and
a measurement portion configured to measure skin resistance between the pair of electrode portions by applying a direct current (DC) voltage to the pair of electrode portions,
wherein the pair of electrode portions has a structure in which drying of the epidermis is naturally performed.

14. The skin resistance measurement device according to claim 13, wherein the measurement portion measures the skin resistance after applying the DC voltage to the pair of electrode portions for a first period of time that is a prescribed period of time in a period before the skin resistance is measured and pauses a process of applying the DC voltage to the pair of electrode portions for a second period of time that is a prescribed period of time.

15. A skin responsiveness assessment method comprising:
a measurement step of measuring skin resistance between a pair of electrode portions provided on an epidermis of a measurement target person and having a structure in which drying of the epidermis is naturally performed; and
an assessment step of assessing responsiveness of the skin to an external environment and a change in life behavior of the measurement target person on the basis of the skin resistance measured in the measurement step.

16. A behavior/emotion determination method comprising:
a measurement step of measuring skin resistance between a pair of electrode portions provided on an epidermis of a measurement target person and having a structure in which drying of the epidermis is naturally performed; and
a determination step of determining behavior or an emotion of the measurement target person on the basis of the skin resistance measured in the measurement step.
